# FASCICULE DE BREVET EUROPEEN

(11) **EP 3 362 176 B1**
(45) Date de publication et mention de la délivrance du brevet: **30.12.2020**
(21) Numéro de dépôt: 16791056.1
(22) Date de dépôt: 12.10.2016
(51) Int. Cl.: B01J 37/08, B01J 37/16, B01J 21/08, B01J 23/00, B01J 23/16, B01J 23/38, B01J 23/70, B01J 35/04, B01J 35/10, B01J 37/02, C03C 11/00, C07C 1/02, C10G 2/00

(54) **NANOPARTICULES METALLIQUES SUPPORTEES SUR UN SUPPORT EN MOUSSE DE VERRE ET UTILISATIONS POUR LA CATALYSE DE RÉACTIONS CHIMIQUES**
AUF GLASSCHAUM GETRÄGTE METALLISCHE NANOPARTIKELN UND DESSEN VERWENDUNG IN KATALYTISCHEN CHEMISCHEN REAKTIONEN
METALLIC NANOPARTICLE SUPPORTED ON A GLASS FOAM AND USES THEREOF FOR CATALYZING CHEMICAL REACTIONS

(30) Priorité: 12.10.2015 FR 1559695
(43) Date de publication de la demande: 22.08.2018
(73) Titulaire: Université de Rennes I, 35000 Rennes (FR); Ecole Nationale Supérieure de Chimie de Rennes, 35708 Rennes Cedex 7 (FR)
(72) Inventeur: BIARD, Pierre-François, 35410 Rennes (FR); COUVERT, Annabelle, 35760 Saint-Gregoire (FR); DENICOURT, Audrey, 35150 Corps-Nuds (FR); ROUCOUX, Alain, 35235 Thorigne Fouillard (FR); LEBULLENGER, Ronan, 35700 Rennes (FR)
(74) Mandataire: Grosset-Fournier, Chantal Catherine
(86) Numéro de dépôt international: PCT/FR2016/052634
(87) Numéro de publication internationale: WO 2017/064418

(56) Documents cités:
- WO-A1-95/35264
- WO-A2-2008/021406
- WO-A2-2015/091816
- US-A- 3 972 721
- US-A1- 2012 028 329
- US-A1- 2014 147 664
- OBREGN ALFARO S ET AL: "Sonochemical deposition of silverTiOnanocomposites onto foamed waste-glass: Evaluation of Eosin Y decomposition under sunlight irradiation", CATALYSIS TODAY, vol. 166, no. 1, 31 juillet 2010 (2010-07-31), pages 166-171, XP028207399, ISSN: 0920-5861, DOI: 10.1016/J.CATTOD.2010.06.028 [extrait le 2010-07-05]
- UNGUREANU S ET AL: "Palladium nanoparticles heterogeneous nucleation within organically grafted silica foams and their use as catalyst supports toward the Suzuki-Miyaura and Mizoroki-Heck coupling reactions", APPLIED CATALYSIS A: GENERAL, ELSEVIER, AMSTERDAM, NL, vol. 390, no. 1-2, 20 décembre 2010 (2010-12-20), pages 51-58, XP027527073, ISSN: 0926-860X [extrait le 2010-10-01]
- UNGUREANU S ET AL: "Syntheses and characterization of new organically grafted silica foams", COLLOIDS AND SURFACES A: PHYSIOCHEMICAL AND ENGINEERING ASPECTS, ELSEVIER, AMSTERDAM, NL, vol. 360, no. 1-3, 5 mai 2010 (2010-05-05) , pages 85-93, XP027009735, ISSN: 0927-7757 [extrait le 2010-02-16]
- FERAL-MARTIN ET AL: "Integrative chemistry toward the first spontaneous generation of gold nanoparticles within macrocellular polyHIPE supports (Au@polyHIPE) and their application to eosin reduction", REACTIVE AND FUNCTIONAL POLYMERS, ELSEVIER, AMSTERDAM, NL, vol. 67, no. 10, 25 septembre 2007 (2007-09-25), pages 1072-1082, XP022267654, ISSN: 1381-5148, DOI: 10.1016/J.REACTFUNCTPOLYM.2007.06.008
- MAXIME LACROIX ET AL: "Silicon carbide foam composite containing cobalt as a highly selective and re-usable FischerTropsch synthesis catalyst", APPLIED CATALYSIS A: GENERAL, ELSEVIER, AMSTERDAM, NL, vol. 397, no. 1, 14 février 2011 (2011-02-14), pages 62-72, XP028193570, ISSN: 0926-860X, DOI: 10.1016/J.APCATA.2011.02.012 [extrait le 2011-02-18]

## Description

La présente invention concerne des nanoparticules métalliques supportées pour la catalyse.

Dans le domaine de la catalyse, la catalyse hétérogène a aujourd'hui une importance capitale. Elle permet notamment d'éviter des étapes de purification du produit issu de la réaction, rendues nécessaires par la contamination du produit par le métal utilisé comme catalyseur.

Un second avantage de la catalyse hétérogène est la possibilité de séparer le catalyseur du milieu réactionnel et de le réutiliser dans la réaction. Il est pour cela nécessaire que le métal jouant le rôle de catalyseur adhère de manière suffisante à son support.

De nombreux supports ont été utilisés en catalyse hétérogène à ce jour.

Les catalyseurs hétérogènes les plus répandus peuvent être classée en trois catégories :
(i) les oxydes métalliques (par exemple Al₂O₃, V₂O₅, TiO₂, Fe₃O₄),
(ii) les métaux de transition supportés,
(iii) les zéolites.

Les métaux de transition supportés représentent la classe la plus intéressante en raison de leur potentiel d'application dans un nombre extrêmement important de procédés catalytiques.

Ces métaux de transition supportés peuvent se présenter sous différentes formes en fonction de la structure du support. Le support peut ainsi être sous la forme de poudres, de pellets ou de monolithes.

Les monolithes ont attiré une attention particulière. Contrairement aux poudres ou aux pellets, les monolithes sont constitués d'un seul bloc, ce qui présente l'avantage de faciliter leur mise en œuvre et de limiter les pertes de charge par rapport à une poudre ou un pellet à porosité et surface spécifique équivalentes.

Les supports de type monolithe ont cependant certains désavantages. Le caractère rectiligne des canaux parallèles composant le monolithe peuvent en effet être à l'origine de mauvais échanges de matière et de chaleur radiaux et une mauvaise distribution du fluide.

Depuis quelques années, on observe une émergence des mousses de céramique dans des applications en catalyse. L'utilisation de ces mousses de céramique à l'échelle industrielle est toutefois freinée par les conditions de préparation assez lourdes et l'emploi indispensable d'une couche d'enduction (« *washcoat* ») préalable au dépôt des espèces métalliques. Cette couche d'enduction, souvent constituée d'oxydes métalliques, comme par exemple Al₂O₃, permet d'augmenter la surface spécifique du matériau support et de faciliter la fixation des espèces métalliques en catalyse. La présence d'une telle couche d'enduction implique cependant une étape supplémentaire dans la préparation du catalyseur.

Des verres poreux peuvent être générés par traitement thermique à des températures de 500 à 760°C pour provoquer une séparation de phases vitreuses suivi d'une dissolution de la phase riche en B₂O₃ et Na₂O. La phase restante est très riche en silice SiO₂. La demande internationale WO 2008/021406 décrit de telles céramiques en tant que support de catalyseurs. Ces céramiques sont préparées en soumettant un verre préparé sans agent porogène à un traitement thermique à une température de l'ordre de 550 °C pour séparer une phase riche en silice et une phase pauvre en silice, suivi d'une attaque acide pour former des pores. Contrairement aux mousses de verre selon la présente invention dont la masse volumique est inférieure à 1, ces matériaux ont une masse volumique de 1,1 g/cm³ à 1,2 g/cm³, traduisant une porosité faible. De plus, les céramiques poreuses décrites dans ce document sont des matériaux 100% cristallin à la différence des mousses de la présente invention qui sont vitreuses ou seulement partiellement cristallisées (jusqu'à 20% de la masse).

Des particules de verre poreux de 50 µm à 500 µm avec une taille moyenne des pores de 20 nm à 100 nm contenant des nanoparticules de Cu et de Pd peuvent être utilisées comme support dans des systèmes catalytiques d'hydrogénation sélective comme décrit dans la demande WO 2015/091816.

Il existe donc aujourd'hui un besoin pour des catalyseurs supportés susceptibles d'être obtenus dans des conditions douces et ne nécessitant pas le dépôt d'une couche d'enduction.

La présente invention vient combler ce besoin.

Un but de l'invention est de fournir un procédé de préparation de nanoparticules supportées simplifié par rapport aux procédés de l'art antérieur.

Un autre but de l'invention est de fournir des nanoparticules métalliques supportées dans lesquelles le métal est en grande partie à l'état d'oxydation 0.

Un autre but de l'invention est de fournir des nanoparticules au degré d'oxydation 0 sur un support essentiellement amorphe, sans couche d'enduction sur ledit support.

L'invention concerne dans un premier aspect un procédé de préparation d'un matériau comprenant :
- un support en mousse de verre,
- des nanoparticules métalliques constituées à au moins 90 % d'un métal à l'état d'oxydation 0 adsorbées sur ledit support,
comprenant une étape de mise en contact de nanoparticules métalliques constituées à au moins 90 % d'un métal à l'état d'oxydation 0 en suspension dans un solvant avec une mousse de verre.

Dans la présente invention, la mousse de verre d'une part et les nanoparticules métalliques constituées à au moins 90 % d'un métal à l'état d'oxydation 0 d'autre part sont préparées séparément l'une de l'autre avant l'étape de mise en contact.

Le procédé selon la présente invention présente ainsi plusieurs avantages sur les procédés de l'art antérieur.
(1) la taille des nanoparticules métalliques constituées à au moins 90 % d'un métal à l'état d'oxydation 0 adsorbées sur la mousse de verre est connue car elle peut être contrôlée lors de leur préparation en utilisant les méthodes de préparation *ad hoc,*
(2) le matériau est prêt à être utilisé, aucune étape de calcination n'étant nécessaire pour former les nanoparticules métalliques constituées à au moins 90 % d'un métal à l'état d'oxydation 0 et/ou les disperser au sein du support.

Le support peut être sous la forme d'un seul bloc ou monolithe, sous la forme de billes ou sous la forme de pellets. Avantageusement, le support est sous la forme d'un seul bloc ou monolithe.

Le volume et la forme du support peuvent être choisis selon l'utilisation désirée. Dans la présente invention, le support a un volume minimum de 0,5 cm³.

Le terme « mousse de verre » désigne, au sens de la présente invention, un matériau macroporeux composé de verre. La mousse de verre est également connue sous les dénominations « verre expansé » et « verre cellulaire ».

La mousse de verre est préparée en mélangeant du verre, avantageusement broyé ou sous forme de billes, avec un agent porogène et en soumettant le mélange ainsi obtenu à une étape de chauffage à une température à laquelle l'agent porogène se décompose pour libérer du gaz et à laquelle la viscosité du verre est suffisamment faible pour que le gaz généré reste emprisonné dans le verre. La porosité est donc créée in situ lors de la décomposition des agents porogènes ou de leurs réactions avec le verre fondu et suffisamment visqueux pour garder prisonnières les bulles de gaz formées (N2, CO₂, ..). Le matériau ainsi obtenu est caractérisé par la présence de pores de diamètre de l'ordre de plusieurs centaines de microns à quelques millimètres. Le matériau se différencie également d'autres supports préparés à partir de verre par une masse volumique apparente très inférieure, témoin de la porosité élevée des matériaux.

Typiquement, la masse volumique des mousses de verre selon la présente invention est inférieure à 1, notamment de 0,15 à 0,8 g/cm³. De préférence, la masse volumique des mousses de verre est de 0,4 à 0,5 g/cm³.

La mousse de verre est caractérisée par le fait que le verre est amorphe ou partiellement cristallisé, contrairement aux céramiques, qui sont des matériaux entièrement cristallins.

Un « verre partiellement cristallisé » est défini au sens de la présente invention, comme un matériau dans lequel la cristallinité est inférieure à 20 %, c'est-à-dire dans lequel au plus 20 % de la masse est sous forme cristalline. Le taux de cristallinité de la mousse de verre peut être déterminé par des méthodes telles que la diffraction aux rayons X.

La mousse de verre selon la présente invention défini par la revendication 6 présente donc l'intérêt de pouvoir être préparée dans des conditions nécessitant un apport d'énergie moindre par rapport à un support cristallin, tel que la céramique.

Les « nanoparticules métalliques constituées à au moins 90 % d'un métal à l'état d'oxydation 0 » se réfèrent à des particules de taille nanométrique, de dimensions comprises entre 1 et 100 nm, constituées par un métal et dont au moins 90 % de ce métal est au degré d'oxydation 0. Un pourcentage de 91 %, 92 %, 93 %, 94 %, 95 %, 96 %, 97 %, 98 %, 99 % ou 100 % dudit au moins un métal au degré d'oxydation 0 est envisageable.

Dans le matériau de l'invention, le métal M au degré d'oxydation 0 est avantageusement un métal de transition du groupe IB, IIB, VA, IVB, VB, VIB, VIIB ou VIIIB.

De manière plus avantageuse, ledit métal de transition M est choisi dans le groupe constitué, du fer, du cobalt, du nickel, du palladium, du ruthénium, du rhodium, de l'or, du platine, du cuivre, de l'argent, du bismuth, du rhénium, du molybdène, de l'iridium, du vanadium, du cadmium et du zinc. De manière préférée, ledit métal de transition M est le rhodium ou le ruthénium.

Dans le procédé selon la présente invention, les nanoparticules métalliques constituées à au moins 90 % d'un métal à l'état d'oxydation 0 ne sont pas générées à partir d'un sel métallique préalablement adsorbé sur le support.

La suspension desdites nanoparticules métalliques constituées à au moins 90 % d'un métal à l'état d'oxydation 0 peut être préparée par réduction d'un sel métallique Mⁿ⁺ ou à partir d'un complexe organométallique dans le solvant, de préférence en présence d'un agent stabilisant, selon des méthodologies décrites dans la littérature. La préparation de nanoparticules métalliques est par exemple décrite dans Comprehensive Organometallic Chemistry III (2007), 12, 71-99 ; Advances in Organometallic Chemistry and Catalysis: The Silver/Gold Jubilee International Conference on Organometallic Chemistry Celebratory Book (ed A. J. L. Pombeiro), John Wiley & Sons, Inc., Hoboken, NJ, USA ; RSC Catalysis Series (2014), 17 (Metal Nanoparticles for Catalysis), 47-82 ; New Journal of Chemistry (2013), 37, (11), 3374-3401 et Comptes Rendus Chimie (2003), 6, (8-10), 1019-1034.

Avantageusement, la suspension est préparée par réduction d'un sel métallique Mⁿ⁺, en raison de la simplicité de mise en œuvre de cette méthode.

Le procédé selon l'invention ne comprend donc pas d'étape de calcination simultanée ou postérieure à l'étape de mise en contact. Par « étape de calcination simultanée ou postérieure à l'étape de mise en contact », on entend, au sens de la présente invention, une étape de chauffage à une température suffisante pour conduire à la réduction du sel métallique adsorbé sur le support, notamment un chauffage à une température supérieure à 250 °C.

Le solvant dans lequel les nanoparticules métalliques constituées à au moins 90 % d'un métal à l'état d'oxydation 0 sont en suspension est choisi parmi les solvants organiques, l'eau ou un mélange de ces solvants.

Avantageusement, le solvant est l'eau ou un alcool, notamment le méthanol, l'éthanol ou le propanol, de préférence le méthanol, ou un mélange eau / alcool, notamment un mélange eau / méthanol ou eau / éthanol, de préférence un mélange eau / méthanol.

La suspension de nanoparticules métalliques constituées à au moins 90 % d'un métal à l'état d'oxydation 0 dans un solvant contient de préférence un agent stabilisant.

Au sens de la présente invention, on entend par « agent stabilisant » un composé capable d'empêcher l'agrégation des nanoparticules métalliques constituées à au moins 90 % d'un métal à l'état d'oxydation 0 générées par la réduction du sel métallique par interactions stériques, électrostatiques ou électrostériques avec lesdites nanoparticules métalliques constituées à au moins 90 % d'un métal à l'état d'oxydation 0.

Le choix de l'agent stabilisant permet notamment de contrôler le diamètre moyen des nanoparticules métalliques constituées à au moins 90 % d'un métal à l'état d'oxydation 0 en suspension dans le solvant.

Des exemples d'agents stabilisants sont les polymères, les liquides ioniques, les ligands phosphorés et/ou soufrés et/ou azotés et/ou oxygénés, les cyclodextrines, les dendrimères, les calixarènes et les tensioactifs.

Dans la présente invention, l'agent stabilisant est avantageusement un tensioactif.

Plus avantageusement, le tensioactif est un ammonium quaternaire, en particulier un N-(hydroxyalkyl)ammonium de formule (I) :

R¹R²R³N⁺(CH₂)ₙ-OH, X⁻

dans laquelle :
R¹ et R², identiques ou différents, avantageusement identiques, représentent un aryle ou alkyle en C₁ à C₆, notamment un alkyle en C₁ à C₃, en particulier un alkyle en C₁ ;
R³ représente un aryle ou alkyle en C₆ à C₂₀, notamment un alkyle en C₁₀ à C₁₈, en particulier un alkyle en C₁₆ ;
n = 2 à 6, notamment 2 à 4, en particulier 2 ou 3 ;
X⁻ est un contre-ion monovalent, notamment choisi parmi F⁻, Cl⁻, Br⁻, I⁻, CF₃SO₃⁻, BF₄⁻, HCO₃⁻, et CH₃SO₃⁻ ou un N-tris-(hydroxyalkyl)ammonium de formule (II) :

   R³N⁺[(CH₂)ₙ-OH]₃, X⁻
dans laquelle R³, X et n sont tels que définis ci-dessus.

Selon un mode de réalisation préféré, l'agent stabilisant est un tensioactif de type ammonium quaternaire, de préférence un N,N-diméthyl-N-cétyl-N-(2-hydroxyéthyl) ammonium.

De manière préférée, l'agent stabilisant est un tensioactif de type ammonium quaternaire, de préférence un N,N-diméthyl-N-cétyl-N-(2-hydroxyéthyl) ammonium et le solvant est l'eau, le méthanol ou un mélange eau / méthanol.

L'utilisation en tant qu'agent stabilisant d'un tensioactif de type ammonium quaternaire, de préférence un N,N-diméthyl-N-cétyl-N-(2-hydroxyéthyl) ammonium et de l'eau, du méthanol ou d'un mélange eau / méthanol, en particulier de l'eau, est particulièrement avantageuse car elle permet d'obtenir des nanoparticules métalliques constituées à au moins 90 % d'un métal à l'état d'oxydation 0 de faible diamètre, notamment compris de 1 à 10 nm, avantageusement de 2 à 8 nm, de préférence environ 5 nm.

L'étape de mise en contact permet l'adsorption des nanoparticules métalliques constituées à au moins 90 % d'un métal à l'état d'oxydation 0 sur le support en mousse de verre.

Ladite étape de mise en contact peut être réalisée par des méthodes telles que l'immersion de la mousse de verre dans une suspension de nanoparticules métalliques constituées à au moins 90 % d'un métal à l'état d'oxydation 0 dans un solvant ou l'imprégnation de la mousse de verre avec la suspension de nanoparticules métalliques constituées à au moins 90 % d'un métal à l'état d'oxydation 0 dans un solvant. L'imprégnation peut notamment être réalisée par diffusion de ladite suspension de nanoparticules à travers les pores de la mousse de verre. Cette imprégnation peut également être réalisée sous vide.

Ladite étape de contact est avantageusement réalisée à température ambiante, c'est-à-dire sans l'utilisation d'une source de chaleur extérieure. La température est donc avantageusement comprise de 0 à 50 °C, avantageusement de 10 à 40 °C, de préférence de 15 à 30 °C, en particulier d'environ 20 °C. Après l'étape de mise en contact, le matériau intermédiaire obtenu est séché afin d'éliminer le solvant dans lequel les nanoparticules étaient en suspension.

L'étape de séchage est mise en œuvre à une température suffisante pour induire l'évaporation dudit solvant. L'étape de séchage peut être mise en œuvre selon des méthodes classiques telles que le séchage dans un four, le séchage sous vide ou par micro-ondes.

Avantageusement, l'étape de séchage est mise en œuvre dans un four, avantageusement à une température de 90 à 200 °C, notamment de 90 à 120 °C, en particulier d'environ 100 °C.

La présente invention concerne donc également un procédé de préparation tel que décrit ci-dessus d'un matériau comprenant :
- un support en mousse de verre,
- des nanoparticules métalliques constituées à au moins 90 % d'un métal à l'état d'oxydation 0 adsorbées sur ledit support,
comprenant les étapes de :
1. mise en contact de nanoparticules métalliques constituées à au moins 90 % d'un métal à l'état d'oxydation 0 en suspension dans un solvant avec une mousse de verre,
2. séchage du matériau obtenu à l'étape (a).

Dans un mode de réalisation avantageux, les étapes de mise en contact (a) et de séchage (b) sont répétées, de préférence jusqu'à adsorption complète des nanoparticules métalliques constituées à au moins 90 % d'un métal à l'état d'oxydation 0 sur la mousse de verre. Cette méthode itérative permet d'augmenter la quantité de nanoparticules métalliques constituées à au moins 90 % d'un métal à l'état d'oxydation 0 adsorbées à la surface de la mousse de verre.

L'adsorption de la totalité des nanoparticules métalliques constituées à au moins 90 % d'un métal à l'état d'oxydation 0 peut être contrôlée visuellement ou par des méthodes de spectroscopie en suivant la décoloration de la suspension de nanoparticules métalliques constituées à au moins 90 % d'un métal à l'état d'oxydation 0 à chaque étape d'imprégnation.

La quantité de nanoparticules adsorbées à la surface de la mousse de verre peut être contrôlée, par exemple, en modifiant la concentration de la suspension de nanoparticules métalliques constituées à au moins 90 % d'un métal à l'état d'oxydation 0. Le pourcentage de nanoparticules métalliques constituées à au moins 90 % d'un métal à l'état d'oxydation 0 adsorbées sur la mousse de verre est ainsi de 0,01 à 5 %, notamment de 0,05 à 2 %, en particulier d'environ 0,1 % en poids du poids total du matériau.

Avantageusement, le procédé selon la présente invention ne comprend pas d'étape pour introduire une couche d'enduction, c'est-à-dire que les nanoparticules métalliques constituées à au moins 90 % d'un métal à l'état d'oxydation 0 sont directement adsorbées sur la mousse de verre.

L'invention concerne donc également un procédé de préparation tel que décrit ci-dessus d'un matériau comprenant :
- un support en mousse de verre,
- des nanoparticules métalliques constituées à au moins 90 % d'un métal à l'état d'oxydation 0 directement adsorbées sur ledit support,
comprenant une étape de mise en contact de nanoparticules métalliques constituées à au moins 90 % d'un métal à l'état d'oxydation 0 en suspension dans un solvant avec une mousse de verre, et ne comprenant pas d'étape visant à introduire une couche d'enduction.

Dans le cas des céramiques, une couche d'enduction ou *washcoat* est systématiquement introduite entre le support en céramique et le catalyseur métallique afin de permettre aux nanoparticules d'adhérer sur le support.

Par « couche d'enduction » ou *washcoat,* on entend au sens de la présente invention un revêtement appliqué à la surface d'un support auquel adhère le catalyseur métallique. Le catalyseur n'est donc pas directement fixé sur le support, mais sur une couche intermédiaire à la surface du support. Il s'agit très souvent d'un matériau dont la porosité élevée permet l'adhérence des nanoparticules au support, tel qu'un oxyde métallique, notamment la gamma-alumine (γ-Al₂O₃). Une couche d'enduction a typiquement une épaisseur comprise de 10 à 200 µm, l'épaisseur optimale étant d'environ 50 µm.

De manière surprenante, les inventeurs ont constaté qu'il n'était pas nécessaire d'appliquer une couche d'enduction entre le support en mousse de verre et les nanoparticules métalliques constituées à au moins 90 % d'un métal à l'état d'oxydation 0.

Le procédé selon la présente invention ne comprend donc pas d'étape visant à introduire, entre la mousse de verre et les nanoparticules métalliques constituées à au moins 90 % d'un métal à l'état d'oxydation 0 une couche supplémentaire, comme par exemple une couche constituée d'oxydes métalliques, destinée à adsorber les nanoparticules métalliques constituées à au moins 90 % d'un métal à l'état d'oxydation 0.

La mousse de verre peut subir, après sa préparation, une étape visant à modifier sa surface.

Ladite étape de modification de la surface peut conduire à une meilleure adsorption des nanoparticules métalliques constituées à au moins 90 % d'un métal à l'état d'oxydation 0 à la surface de la mousse de verre.

L'étape de modification de la surface se différencie d'une étape d'introduction d'une couche d'enduction, en ce sens qu'elle consiste à modifier les fonctions de surface pour améliorer les interactions entre le verre et le métal. Cette fonctionnalisation peut être considérée comme conduisant à la formation d'une strate modifiée à la surface de la mousse de verre d'une épaisseur de l'ordre d'un à quelques microns et inférieure à l'épaisseur d'une couche d'enduction, c'est-à-dire de moins de 10 µm. La présente invention concerne donc également un procédé tel que décrit ci-dessus dans lequel une couche d'épaisseur supérieure à 9 µm n'est pas introduite à la surface de la mousse de verre.

Un premier exemple de fonctionnalisation est le dopage de la mousse de verre avec un ou plusieurs oxydes métalliques tels que TiO₂, Fe₂O₃ ou MnO₂ contribuant à l'activité catalytique.

Un deuxième exemple de fonctionnalisation est la nitruration de la mousse de verre sous atmosphère d'ammoniac afin de modifier les fonctions de surface en remplaçant les atomes d'oxygène par des atomes d'azote ayant une affinité plus importante pour le métal adsorbé.

Un troisième exemple de fonctionnalisation est la cristallisation partielle de la mousse de verre par un traitement thermique. La « cristallisation partielle » au sens de la présente invention s'entend comme une cristallisation d'au plus 20 % de la mousse de verre.

### Préparation de la mousse de verre :

La mousse de verre selon la présente invention peut être préparée selon la méthodologie décrite par exemple dans Journal of Non-Crystalline Solids 356 (2010) 2562-2568.

La mousse de verre est préparée en mélangeant du verre, avantageusement broyé ou sous forme de billes, avec un agent porogène et en soumettant le mélange ainsi obtenu à une étape de chauffage à une température à laquelle l'agent porogène se décompose pour libérer du gaz et à laquelle la viscosité du verre est suffisamment faible pour que le gaz généré reste emprisonné dans le verre.

Au sens de la présente invention, on entend par « agent porogène », un composé capable de générer un gaz lors de sa décomposition.

L'agent porogène est notamment choisi parmi les nitrures (formation de N₂), tels que AlN, Si₃N₄ et TiN, les carbures (formation de CO₂ et CO) tels que SiC, les carbonates (formation de CO₂), tel que le carbonate de calcium (CaCO₃), et le graphite.

La proportion d'agent porogène par rapport à la masse totale du mélange verre / agent porogène est choisie de manière à obtenir une mousse de verre présentant une porosité appropriée. La proportion d'agent porogène est typiquement inférieure ou égale à 5 % en masse par rapport à la masse totale du mélange verre / agent porogène. Avantageusement, la proportion d'agent porogène est de 1 à 5 % en masse par rapport à la masse totale du mélange verre / agent porogène, de préférence de 3 à 5%, en particulier d'environ 4,5 %.

Un agent dopant peut également être utilisé en substitution d'une partie de l'agent porogène.

Au sens de la présente invention, on entend par « agent dopant » un composé capable de favoriser la décomposition de l'agent porogène. L'utilisation d'un agent dopant permet de réduire la quantité d'agent porogène utilisé. Les agents dopants sont notamment des oxydes métalliques, tels que TiO₂ ou Fe₂O₃. Dans le cas du TiO₂, l'agent dopant réagit avec l'agent porogène, tel que AlN selon la réaction suivante :

3TiO₂+4AlN→2Al₂O₃+3TiN+0,5N₂

Le ratio massique entre l'agent porogène et l'agent dopant est de 1 : 1 à 1 : 3, notamment de 1 : 2. Avantageusement, l'agent porogène est AlN, l'agent dopant est TiO₂ et le ratio AlN : TiO₂ est de 1 :2. Dans un mode de réalisation particulier, le mélange est constitué de 95,5 % de verre, 1,5 % d'agent porogène, avantageusement AlN et 3 % d'agent dopant, avantageusement TiO₂, en masse.

Tout type de verre peut en principe être utilisé pour la préparation de la mousse de verre. Il peut par exemple s'agir de verre dit « sale », tel que des résidus d'objets courants comme des ampoules, des tubes cathodiques ou du verre de bouteille, ou encore de pyrex.

La présente invention s'inscrit donc également dans une démarche verte en proposant le recyclage d'un déchet commun (le verre) pour la préparation de matériaux à haute valeur ajoutée. Il est nécessaire, dans le cas d'un verre dit « sale » de contrôler au préalable que le verre ne contient pas ou des traces de plomb, la présence de ce métal étant susceptible de conduire à un empoisonnement du catalyseur.

Des exemples de verre utilisable dans la présente invention, et leur composition en pourcentage massique sont donnés dans le tableau 1 :

**Tableau 1**

| | SiO₂ | B₂O₃ | Al₂O₃ | Na₂O | K₂O | CaO | MgO |
|---|---|---|---|---|---|---|---|
| Verre à récipients | 67 - 70 | | 3-4 | 13-18 | 6-7 | 6-8 | 0-1 |
| Verre flotté | 72,5 | | 1,5 | 13 | 0,3 | 9,3 | 3 |
| Verre de bouteilles | 73 | | 1 | 15 | | 10 | |
| Pyrex® | 80,6 | 12,6 | 2,2 | 4,2 | | 0,1 | 0,05 |
| Verre d'ampoules | 73 | | 1 | 16 | 1 | 5 | 4 |

Avantageusement, le verre utilisé pour la préparation de la mousse de verre a la composition suivante, en pourcentage massique :

| | |
|---|---|
| SiO₂ | 65 à 75 %, avantageusement 70 à 75 %, de préférence 72,5% |
| Na₂O | 10 à 15 %, avantageusement 12 à 15 %, de préférence 13,7 % |
| CaO | 5 à 15 %, avantageusement 8 à 12 %, de préférence 9,8 % |
| MgO | 1 à 5 %, avantageusement 2 à 4 %, de préférence 3,3 % |
| Al₂O₃ | 0,01 à 1 %, avantageusement 0,2 à 0,5 %, de préférence 0,4 % |
| FeO/Fe₂O₃ | 0,01 à 1 %, avantageusement 0,1 à 0,3 %, de préférence 0,2 % |
| K₂O | 0,01 à 0,5 %, avantageusement 0,05 à 0,2 %, de préférence 0,1 %, |

la somme des composants du verre atteignant 100 %.

La température dépend de la nature et de la quantité d'agent porogène, notamment de sa température de décomposition, et de la composition du verre utilisé, notamment de sa viscosité en fonction de la température.

L'étape de chauffage est typiquement réalisée à une température allant de 750 à 1300 °C, notamment de 800 à 900 °C. L'étape de chauffage est typiquement réalisée pendant une période allant de 30 minutes à 8 heures, notamment de 1,5 heure à 4 heures.

Dans des modes de réalisation particuliers, la mousse de verre est préparée à une température de 850 °C pendant 2 heures, à une température de 850 °C pendant 4 heures, à une température de 880 °C pendant 1,5 heure, à une température de 880 °C pendant 2h40 ou à une température de 890 °C pendant 2 heures.

La température requise pour l'étape de chauffage peut être déterminée par analyse thermique différentielle (ATD) et thermo-gravimétrique (ATG) d'un mélange de verre et d'agent porogène, par exemple selon la méthode décrite dans Journal of Non-Crystalline Solids 356 (2010) 2562-2568.

La présente invention concerne également dans un premier mode de réalisation particulier un procédé de préparation tel que décrit ci-dessus d'un matériau comprenant :
- un support en mousse de verre,
- des nanoparticules métalliques constituées à au moins 90 % d'un métal à l'état d'oxydation 0 adsorbées sur ledit support,
comprenant les étapes de :
(a) préparation d'une suspension aqueuse de nanoparticules métalliques constituées à au moins 90 % d'un métal à l'état d'oxydation 0 en présence d'un tensioactif, avantageusement de type ammonium quaternaire, de préférence un N,N-diméthyl-N-cétyl-N-(2-hydroxyéthyl) ammonium dans l'eau,
(b) mise en contact de la suspension de nanoparticules métalliques constituées à au moins 90 % d'un métal à l'état d'oxydation 0 préparée à l'étape a. avec une mousse de verre,
(c) séchage du matériau obtenu à l'étape (b), avantageusement dans une étuve à une température de 90 à 200 °C, notamment de 90 à 120 °C, en particulier d'environ 100 °C,
(d) éventuellement répétition des étapes (b) et (c).

La présente invention concerne également dans un autre mode de réalisation particulier un procédé de préparation tel que décrit ci-dessus d'un matériau comprenant :
- un support en mousse de verre,
- des nanoparticules métalliques constituées à au moins 90 % d'un métal à l'état d'oxydation 0 adsorbées sur ledit support,
comprenant les étapes de :
(a) préparation d'un support en mousse de verre,
(b) préparation d'une suspension de nanoparticules métalliques constituées à au moins 90 % d'un métal à l'état d'oxydation 0 en suspension dans un solvant, avantageusement en présence d'un agent stabilisant,
(c) mise en contact de la mousse de verre obtenue à l'étape (a) avec la suspension de nanoparticules métalliques constituées à au moins 90 % d'un métal à l'état d'oxydation 0 obtenue à l'étape (b),
(d) séchage du matériau obtenu à l'étape (c),
(e) éventuellement, répétition des étapes (c) et (d) jusqu'à adsorption complète des nanoparticules métalliques constituées à au moins 90 % d'un métal à l'état d'oxydation 0 sur le support en mousse de verre.

La présente invention concerne également dans un autre mode de réalisation particulier un procédé de préparation tel que décrit ci-dessus d'un matériau comprenant :
- un support en mousse de verre,
- des nanoparticules métalliques constituées à au moins 90 % d'un métal à l'état d'oxydation 0 adsorbées sur ledit support,
comprenant les étapes de :
(a) préparation d'un support en mousse de verre à partir d'un mélange de :
a. 95 à 98 %, avantageusement 95,5 % en masse de la masse totale du mélange d'un verre de composition suivante, en pourcentage massique :

| | |
|---|---|
| SiO₂ | 65 à 75 %, avantageusement 70 à 75 %, de préférence 72,5% |
| Na₂O | 10 à 15 %, avantageusement 12 à 15 %, de préférence 13,7 % |
| CaO | 5 à 15 %, avantageusement 8 à 12 %, de préférence 9,8 % |
| MgO | 1 à 5 %, avantageusement 2 à 4 %, de préférence 3,3 % |
| Al₂O₃ | 0,01 à 1 %, avantageusement 0,2 à 0,5 %, de préférence 0,4 % |
| FeO/Fe₂O₃ | 0,01 à 1 %, avantageusement 0,1 à 0,3 %, de préférence 0,2 % |
| K₂O | 0,01 à 0,5 %, avantageusement 0,05 à 0,2 %, , de préférence 0,1 %, |

la somme des composants du verre atteignant 100 %,
b. 2 à 5 % d'un agent porogène, et d'un agent dopant, dans un ratio 1 : 2,
à une température de 800 à 900 °C,
(b) préparation d'une suspension de nanoparticules métalliques constituées à au moins 90 % d'un métal à l'état d'oxydation 0 en suspension dans un solvant, avantageusement en présence d'un agent stabilisant,
(c) mise en contact de la mousse de verre obtenue à l'étape (a) avec la suspension de nanoparticules métalliques constituées à au moins 90 % d'un métal à l'état d'oxydation 0 obtenue à l'étape (b),
(d) séchage du matériau obtenu à l'étape (c),
(e) éventuellement, répétition des étapes (c) et (d) jusqu'à adsorption complète des nanoparticules métalliques constituées à au moins 90 % d'un métal à l'état d'oxydation 0 sur le support en mousse de verre.

La présente invention concerne également dans un autre mode de réalisation un procédé de préparation tel que décrit ci-dessus d'un matériau comprenant :
- un support en mousse de verre,
- des nanoparticules métalliques constituées à au moins 90 % d'un métal à l'état d'oxydation 0 adsorbées sur ledit support,
comprenant les étapes de :
(a) préparation d'un support en mousse de verre,
(b) préparation d'une suspension de nanoparticules métalliques constituées à au moins 90 % d'un métal à l'état d'oxydation 0, avantageusement choisi parmi le rhodium et le ruthénium, dans l'eau, le méthanol ou un mélange eau / méthanol, de préférence l'eau, en présence d'un agent stabilisant, avantageusement un tensioactif, de préférence un N,N-diméthyl-N-cétyl-N-(2-hydroxyéthyl) ammonium,
(c) imprégnation de la mousse de verre obtenue à l'étape (a) avec la suspension de nanoparticules métalliques constituées à au moins 90 % d'un métal à l'état d'oxydation 0 obtenue à l'étape (b),
(d) séchage du matériau obtenu à l'étape (c), notamment à une température de 90 à 120 °C, de préférence environ 100 °C,
(e) éventuellement, répétition des étapes (c) et (d) jusqu'à adsorption complète des nanoparticules métalliques constituées à au moins 90 % d'un métal à l'état d'oxydation 0 sur le support en mousse de verre.

La présente invention concerne dans un mode de réalisation préféré, un procédé de préparation tel que décrit ci-dessus d'un matériau comprenant :
- un support en mousse de verre,
- des nanoparticules métalliques constituées à au moins 90 % d'un métal à l'état d'oxydation 0 adsorbées sur ledit support,
comprenant les étapes de :
(a) préparation d'un support en mousse de verre à partir d'un mélange de :
a. 95 à 98 %, avantageusement 95,5 % en masse de la masse totale du mélange d'un verre de composition suivante, en pourcentage massique :

| | |
|---|---|
| SiO₂ | 65 à 75 %, avantageusement 70 à 75 %, de préférence 72,5% |
| Na₂O | 10 à 15 %, avantageusement 12 à 15 %, de préférence 13,7 % |
| CaO | 5 à 15 %, avantageusement 8 à 12 %, de préférence 9,8 % |
| MgO | 1 à 5 %, avantageusement 2 à 4 %, de préférence 3,3 % |
| Al₂O₃ | 0,01 à 1 %, avantageusement 0,2 à 0,5 %, de préférence 0,4 % |
| FeO/Fe₂O₃ | 0,01 à 1 %, avantageusement 0,1 à 0,3 %, de préférence 0,2 % |
| K₂O | 0,01 à 0,5 %, avantageusement 0,05 à 0,2 %, de préférence 0,1 %, |

la somme des composants du verre atteignant 100 %,
b. 3 à 5 % d'un agent porogène, et d'un agent dopant, dans un ratio 1 : 2, à une température de 800 à 900 °C,
(b) préparation d'une suspension de nanoparticules métalliques constituées à au moins 90 % de rhodium(0) ou de ruthénium(0) dans l'eau ou le méthanol en présence d'un tensioactif, de préférence un N,N-diméthyl-N-cétyl-N-(2-hydroxyéthyl) ammonium,
(c) imprégnation de la mousse de verre obtenue à l'étape (a) avec la suspension de nanoparticules métalliques constituées à au moins 90 % d'un métal à l'état d'oxydation 0 obtenue à l'étape (b),
(d) séchage du matériau obtenu à l'étape (c) à une température de 90 à 120 °C, de préférence environ 100 °C,
(e) éventuellement, répétition des étapes (c) et (d) jusqu'à adsorption complète des nanoparticules métalliques constituées à au moins 90 % d'un métal à l'état d'oxydation 0 sur le support en mousse de verre.

Dans un second aspect, la présente invention concerne également un matériau susceptible d'être obtenu par le procédé tel que décrit ci-dessus.

Dans un troisième aspect, la présente invention concerne un matériau comprenant :
- un support en mousse de verre,
- des nanoparticules métalliques constituées à au moins 90 % d'un métal à l'état d'oxydation 0 adsorbées sur ledit support,
ladite mousse de verre présentant une porosité **ouverte** d'au moins 75 % par rapport à la porosité totale, avantageusement d'au moins 80 %, de préférence au moins 85 %.

Le matériau selon la présente invention peut être sous la forme d'un monolithe, de granulés ou de billes. Avantageusement, le matériau est un monolithe.

La mousse de verre peut être caractérisée par trois types de porosité :
(1) la **porosité totale** est définie comme la proportion du volume total occupée par les pores.
(2) la **porosité fermée** est définie comme la proportion de pores qui ne sont pas reliés entre eux ou avec l'extérieur. Ces pores ne sont pas accessibles par les fluides ou les gaz extérieurs.
(3) la **porosité ouverte** est définie comme la proportion de pores reliés entre eux et accessibles aux gaz et aux fluides extérieurs.

Avantageusement, le matériau selon la présente invention ne contient pas de couche d'enduction.

La présente invention concerne donc en particulier un matériau comprenant :
- un support en mousse de verre,
- des nanoparticules métalliques constituées à au moins 90 % d'un métal à l'état d'oxydation 0 directement adsorbées sur ledit support,
ladite mousse de verre présentant une porosité **ouverte** d'au moins 75 % par rapport à la porosité totale, avantageusement d'au moins 80 %, de préférence au moins 85 %.

La présente invention concerne également en particulier un matériau défini par la revendication 1, ledit matériau comprenant :
- un support en mousse de verre,
- des nanoparticules métalliques constituées à au moins 90 % d'un métal à l'état d'oxydation 0 directement adsorbées sur ledit support,
ladite mousse de verre présentant une porosité **ouverte** d'au moins 75 % par rapport à la porosité totale, avantageusement d'au moins 80 %, de préférence au moins 85 %, ne comprenant pas de couche d'enduction.

La mousse de verre du matériau selon la présente invention est également caractérisée par une porosité totale d'au moins 60 %, avantageusement d'au moins 70 %, de manière plus avantageuse d'au moins 75 % et de préférence d'au moins 80 %.

La porosité ouverte et la porosité totale ont été déterminées selon les méthodes décrites dans les parties correspondantes de la section « mesure des paramètres » de la présente description.

Au-delà de 95 % de porosité ouverte, les nanoparticules métalliques constituées à au moins 90 % d'un métal à l'état d'oxydation 0 ne peuvent pas être imprégnées sur la mousse de verre. De plus, la mousse de verre devient trop fragile. Avantageusement, la mousse de verre dudit matériau a donc une porosité ouverte de 75 à 95 %, avantageusement de 80 à 95 %, de préférence de 85 à 95 %.

Dans les matériaux selon la présente invention, le diamètre moyen des pores de la mousse de verre dₚ dépend de la composition du verre, de la nature et de la proportion de l'agent porogène et éventuellement de la présence ou non d'un agent dopant, ainsi que de la température de préparation de la mousse de verre. Le diamètre moyen des pores peut donc être contrôlé en choisissant ces paramètres.

Le diamètre moyen dₚ des pores dans les mousses de verre selon la présente invention est de 0,01 mm à 1 mm, avantageusement de 0,2 à 0,8 mm, notamment de 0,4 à 0,7 mm. Le diamètre des pores peut être déterminé selon différentes méthodes telles que l'analyse d'une image de la surface d'une mousse de verre et d'un référentiel de mesure permettant le calibrage (par exemple un pied à coulisses) et traitement d'images.

Dans le cadre de la présente invention, le diamètre des pores est déterminé par traitement d'images grâce au logiciel ImageJ, développé par le *National Institut of Health* selon la méthode décrite dans la section « Mesure des paramètres ».

La composition de la mousse de verre dépend de la composition du verre utilisé pour sa préparation et de l'agent porogène et de l'éventuellement agent dopant, qui entrent dans la composition de la mousse de verre.

Avantageusement, la mousse de verre est composée des éléments suivants, en pourcentage massique :

| | |
|---|---|
| SiO₂ | 60 à 77 %, |
| Na₂O | 3 à 17 %, |
| CaO | de 4 à 10 % ou B₂O₃ de 9 à 13 % |

La surface de la mousse de verre dans le matériau selon la présente invention peut être modifiée. Des exemples de modifications sont la nitruration, la cristallisation partielle ou le dopage de la mousse de verre avec un ou plusieurs oxydes métalliques tels que TiO₂, Fe₂O₃ ou MnO₂ contribuant à l'activité catalytique.

Le métal des nanoparticules métalliques constituées à au moins 90 % d'un métal à l'état d'oxydation 0 est avantageusement un métal de transition du groupe IB, IIB, VA, IVB, VB, VIB, VIIB ou VIIIB, de préférence choisi dans le groupe constitué de fer, cobalt, nickel, palladium, ruthénium, or, platine, cuivre, argent, bismuth, rhénium, molybdène, iridium, vanadium, cadmium et zinc.

Dans un mode de réalisation particulier, le métal constituant lesdites nanoparticules métalliques constituées à au moins 90 % d'un métal à l'état d'oxydation 0 est un métal du groupe VIIIB, notamment le palladium, le ruthénium ou le rhodium. De préférence, le métal est le rhodium ou le ruthénium.

De manière avantageuse, les nanoparticules métalliques constituées à au moins 90 % d'un métal à l'état d'oxydation 0 ont un diamètre de 1 à 10 nm, avantageusement de 2 à 8 nm, de préférence d'environ 5 nm.

La faible taille des nanoparticules offre une surface spécifique et un nombre de sites actifs important qui permettent d'obtenir des performances élevées lors de la mise en œuvre de réactions de catalyse.

Dans la présente invention, le diamètre moyen des nanoparticules est mesuré avec le logiciel imageJ, selon la méthode décrite pour la détermination du diamètre moyen des pores dans la section « Mesure des paramètres », l'image utilisée étant celle d'une suspension de nanoparticules métalliques constituées à au moins 90 % d'un métal à l'état d'oxydation 0 dans un solvant obtenue par microscopie électronique à balayage ou par microscopie électronique à transmission. L'analyse du diamètre moyen des nanoparticules peut également être réalisée avec le logiciel SCION, développé par le *National Institute of Health.*

Le pourcentage de nanoparticules métalliques constituées à au moins 90 % d'un métal à l'état d'oxydation 0 adsorbées à la surface de la mousse de verre dépend principalement de la concentration en nanoparticules de la suspension utilisée pour la préparation du matériau. Il existe également pour chaque mousse de verre un seuil maximum d'adsorption, dépendant notamment de la porosité de la mousse de verre et du diamètre moyen des pores.

Le pourcentage de nanoparticules métalliques constituées à au moins 90 % d'un métal à l'état d'oxydation 0 est choisi de manière à obtenir une activité catalytique satisfaisante. Le pourcentage maximum de nanoparticules métalliques constituées à au moins 90 % d'un métal à l'état d'oxydation 0 doit également prendre en compte des facteurs économiques.

Avantageusement, le pourcentage de nanoparticules métalliques constituées à au moins 90 % d'un métal à l'état d'oxydation 0 adsorbées à la surface de la mousse de verre dans les matériaux selon la présente invention est de 0,01 à 5 %, avantageusement de 0,05 à 2 %, de préférence d'environ 0,1 %. Dans un premier mode de réalisation particulier, la présente invention concerne un matériau tel que décrit ci-dessus, comprenant :
- un support en mousse de verre, présentant une porosité ouverte d'au moins 75 %, avantageusement d'au moins 80 %, de préférence au moins 85 %, une porosité totale d'au moins 60 %, avantageusement d'au moins 70 %, de manière plus avantageuse d'au moins 75 % et de préférence d'au moins 80 % et un diamètre moyen des pores dₚ de 0,01 mm à 1 mm, avantageusement de 0,2 à 0,8 mm, notamment de 0,4 à 0,7 mm,
- des nanoparticules métalliques constituées à au moins 90 % d'un métal à l'état d'oxydation 0.

Dans un deuxième mode de réalisation particulier, la présente invention concerne un matériau tel que décrit ci-dessus, comprenant :
- un support en mousse de verre monolithique, présentant une porosité ouverte d'au moins 75 %, avantageusement d'au moins 80 %, de préférence au moins 85 %,
- 0,01 à 5 %, avantageusement de 0,05 à 2 %, de préférence d'environ 0,1 % de nanoparticules métalliques constituées à au moins 90 % d'un métal du groupe IB, IIB, VB, VIB, VIIB ou VIIIB, de préférence le rhodium, le ruthénium ou le palladium, à l'état d'oxydation 0, de diamètre moyen de 1 à 10 nm, avantageusement de 2 à 8 nm, de préférence environ 5 nm adsorbées sur ledit support.

Dans un troisième mode de réalisation particulier, la présente invention concerne un matériau tel que décrit ci-dessus, comprenant :
- un support en mousse de verre monolithique, présentant une porosité ouverte d'au moins 75 %, avantageusement d'au moins 80 %, de préférence au moins 85 %, une porosité totale d'au moins 60 %, avantageusement d'au moins 70 %, de manière plus avantageuse d'au moins 75 % et de préférence d'au moins 80 % et un diamètre moyen des pores dₚ de 0,01 mm à 1 mm, avantageusement de 0,2 à 0,8 mm, notamment de 0,4 à 0,7 mm,
- 0,01 à 5 %, avantageusement de 0,05 à 2 %, de préférence d'environ 0,1 % de nanoparticules métalliques constituées à au moins 90 % d'un métal à l'état d'oxydation 0, de diamètre moyen de 1 à 10 nm, avantageusement de 2 à 8 nm, de préférence environ 5 nm adsorbées sur ledit support.

Dans un quatrième mode de réalisation particulier, la présente invention concerne un matériau tel que décrit ci-dessus, comprenant :
- un support en mousse de verre monolithique, présentant une porosité ouverte d'au moins 80 %, de préférence au moins 85 %, une porosité totale d'au moins 75 %, de préférence d'au moins 80 % et un diamètre moyen des pores dₚ de 0,2 à 0,8 mm, notamment de 0,4 à 0,7 mm,
- 0,05 à 2 %, de préférence d'environ 0,1 % de nanoparticules métalliques constituées à au moins 90 % d'un métal à l'état d'oxydation 0, avantageusement un métal du groupe VIIIb, notamment choisi parmi le ruthénium et le rhodium, de diamètre moyen de 2 à 8 nm, de préférence environ 5 nm adsorbées sur ledit support.

Dans un cinquième mode de réalisation, la présente invention concerne un matériau tel que décrit ci-dessus, comprenant :
(a) un support monolithique en mousse de verre comprenant en pourcentage massique :

| | |
|---|---|
| SiO₂ | 60 à 77 %, |
| Na₂O | 3 à 17 %, |
| CaO | de 4 à 10 % ou B₂O₃ de 9 à 13 %, |

(b) des nanoparticules constituées d'au moins 90 % de Rh(0) de diamètre moyen de 2 à 8 nm, de préférence environ 5 nm supportées sur ledit support monolithique, ladite mousse de verre présentant une porosité **ouverte** d'au moins 75 %, avantageusement d'au moins 80 %, de préférence au moins 85 %,
une porosité totale d'au moins 60 %,
le diamètre moyen des pores de la mousse de verre dₚ étant de 0,4 à 0,7 mm.

L'invention concerne également l'utilisation d'un matériau tel que défini ci-dessus en tant que catalyseur.

La présente invention concerne l'utilisation d'un matériau tel que défini ci-dessus pour la dégradation de polluants en présence d'ozone. Avantageusement, les nanoparticules métalliques constituées à au moins 90 % d'un métal à l'état d'oxydation 0 du matériau sont constituées de ruthénium.

Les matériaux selon la présente invention peuvent donc être avantageusement utilisés pour la dépollution des eaux usées, par exemple dans les stations de traitement des eaux ou d'épuration.

La présente invention concerne également l'utilisation d'un matériau tel que défini ci-dessus pour les réactions de réductions en phase gazeuse en présence d'H₂ telles que l'hydrogénation d'alcènes, d'alcynes ou d'arènes et la réaction tandem déshalogénation/hydrogénation. Avantageusement, les nanoparticules métalliques constituées à au moins 90 % d'un métal à l'état d'oxydation 0 du matériau sont constituées de rhodium, de palladium, de nickel, d'iridium ou de ruthénium, de préférence de rhodium.

La présente invention concerne également l'utilisation d'un matériau tel que défini ci-dessus pour les réactions d'oxydations en phase gazeuse en présence d'O₂.

La présente invention concerne également en particulier l'utilisation d'un matériau tel que défini ci-dessus pour la préparation d'ammoniac NH₃ en phase gazeuse à partir de N₂ et de H₂. Avantageusement, les nanoparticules métalliques constituées à au moins 90 % d'un métal à l'état d'oxydation 0 sont constituées de fer ou de ruthénium.

La présente invention concerne également en particulier l'utilisation d'un matériau tel que défini ci-dessus pour les réactions en phase gazeuse en présence de CO, telle que la réaction de Fischer-Tropsch et les réactions de carbonylations. Avantageusement, les nanoparticules métalliques constituées à au moins 90 % d'un métal à l'état d'oxydation 0 du matériau sont constituées de ruthénium, de palladium, de fer ou de cobalt.

La présente invention concerne également l'utilisation d'un matériau tel que défini ci-dessus pour les réactions de carboxylations en phase gazeuse en présence de CO₂ ou la réduction de N₂O en N₂ ou NH₃. Avantageusement, les nanoparticules métalliques du matériau sont constituées de palladium, de nickel, de fer ou de cobalt.

De manière avantageuse, la présente invention concerne l'utilisation d'un matériau tel que défini ci-dessus, comprenant avantageusement des nanoparticules de Fe(0), Ir(0), Ru(0), Rh(0), de Pd(0) ou de Pt(0), de préférence de Rh(0), Ru(0), pour l'hydrogénation d'alcènes, d'alcynes ou d'arènes et la réaction tandem déshalogénation/hydrogénation.

La présente invention concerne également un réacteur catalytique comprenant un matériau tel que défini dans l'invention.

Ledit réacteur peut être un réacteur en batch ou un réacteur en continu.

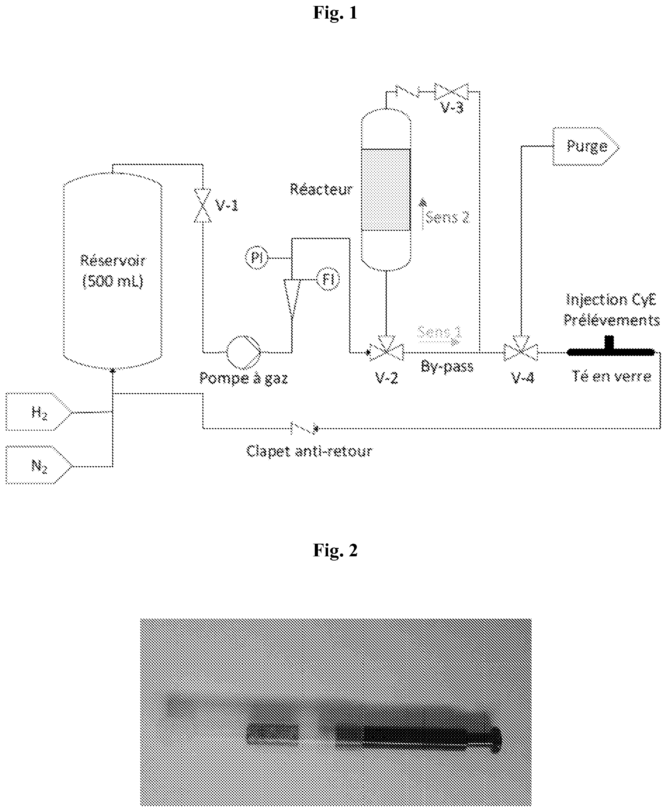

Les matériaux selon la présente invention, en raison de leur structure poreuse permettent un passage des fluides et des gaz au sein du matériau. De plus les matériaux pouvant être sous la forme de monolithes, ils sont particulièrement avantageux dans des réacteurs continu, en particulier dans des réacteurs à lit fixe. Un exemple de réacteur continu est représenté schématiquement dans la figure 1.

Pour les procédés en phase gazeuse, un montage similaire à celui de la figure 1 est particulièrement adapté.

La mise en œuvre d'un procédé dans un réacteur à lit fixe consiste en un remplissage du circuit avec le ou les gaz (O₂, N₂ et H₂, CO, CO et H₂, ou CO₂) et le réactif.

Lors du passage dans le matériau, le réactif et les gaz entrent en contact avec les nanoparticules métalliques pour donner lieu à la réaction et à la formation du produit attendu.

La présente invention concerne donc également l'utilisation d'un réacteur comprenant un matériau ci-dessus pour la dégradation de polluants en présence d'ozone, les réactions de réductions en phase gazeuse en présence d'H₂ telles que l'hydrogénation d'alcènes, d'alcynes ou d'arènes et la réaction tandem déshalogénation/hydrogénation, les réactions d'oxydations en phase gazeuse en présence d'O₂, la préparation d'ammoniac NH₃ en phase gazeuse à partir de N₂ et de H₂, les réactions en phase gazeuse en présence de CO, telle que la réaction de Fischer-Tropsch et les réactions de carbonylations, les réactions de carboxylations en phase gazeuse en présence de CO₂, ou la décomposition du N₂O en N₂, éventuellement en présence de H₂ ou CO.
La Figure 1 est une représentation schématique d'un réacteur à lit fixe contenant un matériau tel que défini dans la présente invention.
La Figure 2 représente un système à piston utilisé pour l'imprégnation d'une mousse de verre.
La Figure 3 représente la concentration en cyclohexane (CyA, carrés) et cyclohéxène (CyE, losanges) dans la réaction d'hydrogénation en fonction du temps avec la mousse **Rh/MV1.** Les courbes sont celles d'une cinétique théorique d'ordre 1. La superposition des points expérimentaux et de la courbe théorique confirment une cinétique d'ordre 1 pour la mousse **Rh/MV1** dans la réaction d'hydrogénation.
La Figure 4 représente le montage utilisé pour l'étude de la dégradation de l'ozone.
La Figure 5 représente la concentration en ozone en fonction du temps en présence du mélange A.
La Figure 6 représente la concentration en ozone en fonction du temps en présence du mélange B.
La Figure 7 représente la concentration en ozone en fonction du temps en présence du mélange C.
La Figure 8 représente la concentration en ozone en fonction du temps en présence du mélange D.
La Figure 9 représente la concentration en ozone en fonction du temps en présence du mélange E.
La figure 10 représente la concentration en pCBA au cours du temps en présence du mélange F.

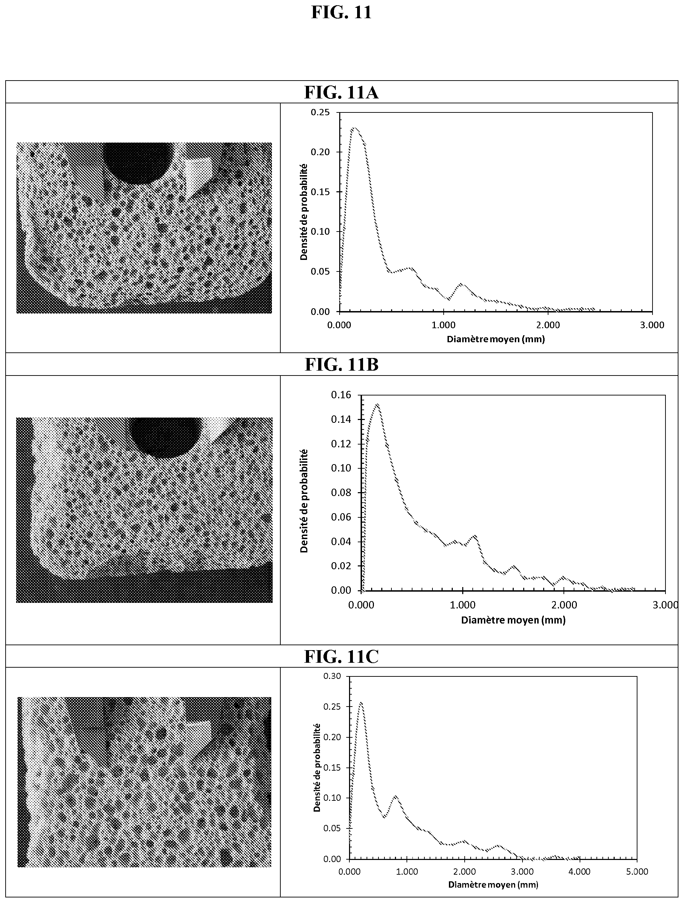

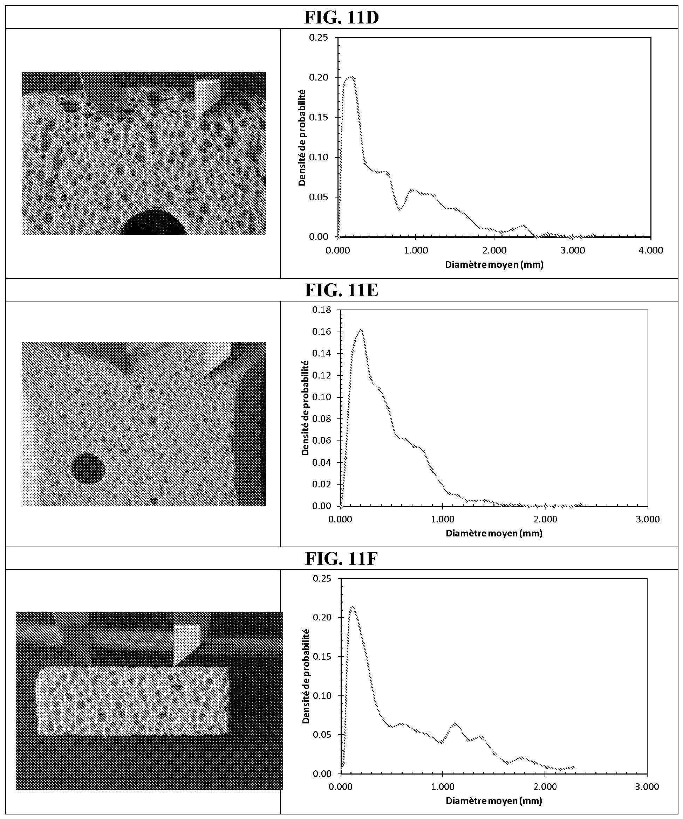

Les Figures 11A à 11F montrent dans le cadre de gauche la photographie de mousses de verre selon la présente invention (l'écartement du pied à coulisses étant de 25 mm) et dans le cadre de droite la densité de probabilités (en ordonnées) en fonction du diamètre des pores (abscisse) telle que déterminée avec le logiciel ImageJ utilisée pour déterminer le diamètre moyen des pores.

Les mousses de verre ont été préparées dans des conditions de traitement thermique identiques et diffèrent seulement par la nature et la proportion de l'agent porogène.

| | Type de verre | Agents porogènes % wt | Traitement thermique |
|---|---|---|---|
| Fig. 11A | Billes de verre (silicate sodocalcique blanc) | 2% AlN + 2% TiO₂ | Montée à 880°C en 2h puis maintien à 880°C pdt 2h puis refroidissement |
| Fig. 11B | Billes de verre (silicate sodocalcique blanc) | 1% AlN +1%TiO₂ + 1% Fe₂O₃ | Montée à 880°C en 2h puis maintien à 880°C pdt 2h puis refroidissement |
| Fig. 11C | Billes de verre (silicate sodocalcique blanc) | 2% AlN + 2% TiO₂ | Montée à 880°C en 2h puis maintien à 880°C pdt 2h puis refroidissement |
| Fig. 11D | Billes de verre (silicate sodocalcique blanc) | 2% AlN + 1% TiO₂ | Montée à 880°C en 2h puis maintien à 880°C pdt 2h puis refroidissement |
| Fig. 11E | Billes de verre (silicate sodocalcique blanc) | 3% AlN | Montée à 880°C en 2h puis maintien à 880°C pdt 2h puis refroidissement |
| Fig. 11F | Billes de verre (silicate sodocalcique blanc) | 1,5% AlN + 1,5% TiO₂ | Montée à 880°C en 2h puis maintien à 880°C pdt 2h puis refroidissement |

### MESURE DES PARAMETRES :

### METHODE DE DETERMINATION DE LA POROSITE OUVERTE :

Le pourcentage de porosité ouverte, au sens de l'invention, correspond au volume occupé par les pores communiquant entre eux et/ou avec le milieu extérieur par rapport à la porosité totale.

Ladite porosité **ouverte** de la mousse de verre peut être déterminée selon l'équation : ${%P}_{ouverte} = \left(1-\frac{{ρ}_{app}}{{ρ}_{pyc}}\right) \times 100 ,$ où :
*ρₐₚₚ* est la masse volumique apparente déterminée par le ratio entre la masse de la mousse et son volume total incluant les pores
*ρ_{pyc}* est la masse volumique mesurée par pycnométrie à l'hélium.

Dans la présente invention, la masse volumique apparente ρₐₚₚ est calculée selon la méthode suivante :
- un morceau de mousse de verre est découpé,
- son volume V est déterminé précisément. Par exemple, le morceau de mousse de verre est un parallélépipède dont les dimensions extérieures sont mesurées à l'aide d'un pied à coulisse,
- le morceau de mousse de verre, nettoyé afin d'éliminer les éventuelles poussières de verre, est pesé pour déterminer sa masse m.

On note ρₐₚₚ la valeur de la masse volumique apparente calculée par la formule : ρₐₚₚ= m / V.

Le principe du pycnomètre à Hélium est de déterminer la masse volumique de la mousse de verre sans tenir compte du volume des pores ouverts. Ainsi, connaissant la masse volumique apparente et la masse volumique réelle *ρ_{pyc},* les pourcentages de porosité ouverte et fermée peuvent être déterminés. Dans la présente invention, la masse volumique réelle *ρ_{pyc}* est mesurée par pycnométrie à l'Hélium selon la méthode suivante :
Le pycnomètre utilisé est un pycnomètre de marque AccuPyc 1330 V2.
- l'échantillon est introduit dans le pycnomètre et le pycnomètre est purgé au moins cinq fois,
- le gaz est confiné (à la pression P₁) dans une cellule de volume (V_{c}) connu.
- l'hélium est ensuite libéré dans un volume de détente (V₂), on obtient une pression P₂.
On note Pₐ la pression atmosphérique.

Le volume (Vₑ) de la masse connue (M) de l'échantillon est déterminé selon la loi de Mariotte (1) : $\left({P}_{1}-{P}_{a}\right) \times {V}_{1} = \left({P}_{2}-{P}_{a}\right) \times {V}_{2}$ (1)

Sachant que : ${V}_{1} = {V}_{c} - {V}_{e} ,$ on en déduit : ${V}_{e} = {V}_{c} - \frac{\left({P}_{2}-{P}_{a}\right) \times {V}_{2}}{{P}_{1} - {P}_{a}}$

La masse volumique réelle ρ_{pyc} se déduit ensuite de la relation ρ_{pyc} = M / Vₑ.

### METHODE DE DETERMINATION DE LA POROSITE TOTALE

La porosité totale Pₜₒₜ est déterminée selon l'équation : ${%P}_{tot} = \left(1-\frac{{ρ}_{app}}{{ρ}_{verre}}\right) \times 100$ dans laquelle *ρₐₚₚ* est la masse volumique apparente déterminée par le ratio entre la masse de la mousse et son volume total incluant les pores, selon la méthode décrite ci-dessus.

*ρᵥₑᵣᵣₑ* est la masse volumique du verre utilisé pour préparer la mousse de verre.

Par « verre utilisé pour préparer la mousse de verre », on entend au sens de la présente invention le verre brut n'ayant pas encore subi d'étape visant à introduire une porosité. La valeur *ρᵥₑᵣᵣₑ* est une valeur déterminée par le fournisseur du verre en question.

### METHODE DE DETERMINATION DU DIAMETRE MOYEN DES PORES

Le logiciel ImageJ permet d'effectuer une analyse du nombre et de la taille de tous les pores d'une zone choisie. Une fois le dénombrement des pores obtenus, ceux-ci sont regroupés par catégories de taille. Le nombre de catégories est égal à la racine carrée du nombre de pores. Une moyenne arithmétique est ensuite effectuée, afin d'obtenir la taille moyenne des pores de la mousse étudiée : ${d}_{P} = \frac{{\sum }_{i = 1}^{j} \frac{{d}_{i} + {d}_{i + 1}}{2} \times {n}_{i}}{n}$ où dᵢ et dᵢ₊₁ représentent les bornes de chaque intervalle, nᵢ le nombre de pores par intervalles, n le nombre de pores totaux et j le nombre d'intervalles.

La méthode utilisée est la suivante :
- une image de la surface d'une mousse de verre et d'un référentiel de mesure permettant le calibrage (par exemple un pied à coulisses) est convertie en une image en niveaux de gris,
- l'échelle de l'image est calibrée :
   ∘ *Analyze> SetScale> Click to Remove Scale* afin d'annuler l'échelle définie dans de précédentes analyses,
   ∘ la commande *"Straight"* est utilisée pour déterminer l'échelle, le segment est placé aux extrémités du référentiel de mesure,
   ∘ la taille du référentiel est renseignée dans *Analyze>SetScale> "known distance",* ainsi que l'unité dans *"unit oflenght",*
- un duplicata de la section de l'image à étudier est effectué. La zone d'étude est sélectionnée à l'aide de l'outil *"rectangular",* et *Image>Duplicate.*
- la taille minimale et maximale en pixels des pores est déterminée : sélectionner *Process>FFT>BandpassFilter* et définir les paramètres suivants :
   ∘ taille minimale du filtre : 3/8,
   ∘ taille maximale du filtre : 200,
   ∘ *Suppress stripes : None,*
   ∘ *Tolerance of direction* : *5,*
   ∘ sélectionner *"Autoscale afterfiltering"* et *"saturate image",*
   ∘ désélectionner *"Display filter",*
   ∘ presser OK.
- la définition du logiciel est ensuite ajustée :
   ∘ sélectionner *Image>Adjust>Treshold,*
   ∘ l'image initiale (duplicata) est comparée avec l'image sous *"treshold".*
   ∘ on fait apparaitre les pores visuellement différenciables sur la photo et on fait apparaitre les plus petits pores,
   ∘ la mesure des particules est effectuée. *Analyze>Analyze Particles Size: 0-infinity Show: -Outlines* afin de faire une fenêtre montrant les résultats de l'analyse et les pores sélectionnés par le logiciel.

### EXEMPLES

### Exemple 1 : Préparation de la mousse de verre

### Exemple 1.1 :

477,5 g de billes de verre « sales » (95,5% de la masse totale de mousse), 15g de AlN (3% de mₜₒₜₐₗₑ) et 7,5g d'agent dopant TiO₂ (1,5% de mₜₒₜₐₗₑ) sont mélangés dans un récipient introduit dans un four. Trois mousses ont été préparées à des températures différentes et des temps de cuisson différents afin de faire varier la porosité ouverte :

| **Mousse de verre** | **Temps (h)** | **Température (°C)** | **Porosité ouverte (%)** |
|---|---|---|---|
| **MV1** | 2 | 890 | 85 |
| **MV2** | 1,5 | 880 | 83 |
| **MV3** | 4 | 850 | 77 |

### Exemple 1.2 :

Selon la même méthode, une quatrième mousse de verre est préparée à partir de 200 g de billes de verre « sales », 2 % en poids d'AlN et 1 % en poids de TiO₂ à une température de 850 °C pendant 1h40.

Les caractéristiques de la mousse de verre **MV4** ont ensuite été déterminées :

| *ρₐₚₚ* | *ρ_{pyc}* | %Porosité ouverte | %Porosité fermée | %Porosité totale |
|---|---|---|---|---|
| 0,39 | 2,06 | 81 | 3 | 84 |

### Exemple 1.3 :

La mousse de verre **MV5** est préparée à partir de billes de verre « sales » (95,5%), 1,5 % en poids d'AlN et 3 % en poids de TiO₂ à une température de 880 °C pendant 2h40.

### Exemple 1.4 : Nitruration d'une mousse de verre

Un échantillon de mousse de verre selon l'exemple 1.1 est placé dans un creuset en céramique, dans un four en céramique. Sous flux d'azote, une température de 750 °C est atteinte en 1h15. Après 35 min à 750 °C, le flux d'azote est remplacé par un flux d'ammoniac. Le chauffage est maintenu 24 h sous flux d'ammoniac puis la mousse est naturellement refroidie, en 2h environ, sous flux d'azote.

### Exemple 2 : Préparation des suspensions de nanoparticules

### Exemple 2.1 : Préparation d'une suspension aqueuse nanoparticules de ruthénium(0) :

10 mL d'une solution aqueuse contenant 49,7 mg (1eq, 1,9·10⁻⁴ mol) de RuCl₃,3H₂O et 37 mL d'une solution aqueuse contenant 133 mg (2 eq, 3,8·10⁻⁴ mol) de N,N-diméthyl-N-cétyl-N-(2-hydroxyéthyl) ammonium sont ajoutées, sous agitation, à 3 mL d'une solution aqueuse de NaBH4 (2,5 eq, 4,75·10⁻⁴ mol, 18 mg). Le mélange est maintenu sans agitation.

### Exemple 2.2 : Préparation d'une suspension aqueuse de nanoparticules de rhodium(0) :

Dans un ballon de 25 mL, 7,95 mg (3,02·10⁻⁵ mol, 1 éq) de RhCl₃ dissous dans 2 mL d'eau sont introduits et agités pendant quelques minutes. 25,6 mg de N,N-diméthyl-N-cétyl-N-(2-hydroxyéthyl) ammonium (7,3·10⁻⁵ mol, 2,41 éq) sont solubilisés dans 7,5 mL d'eau (1·10⁻³ mol·L⁻¹) puis 4,30 mg de NaBH4 (1,14·10⁻⁴ mol, 3,77 éq) dans 0,5 mL d'eau sont ajoutés à la solution de N,N-diméthyl-N-cétyl-N-(2-hydroxyéthyl) ammonium. Rapidement, ce mélange est ajouté à la solution du sel métallique. Un changement de couleur de la solution (du rouge au noir) est observé. La suspension est maintenue sous agitation. Elle peut être stable pendant plusieurs mois. La concentration en Rh(0) est donc de 3,02·10⁻³ mol·L⁻¹.

### Exemple 3 : Préparation de nanoparticules adsorbées sur la mousse de verre

### Exemple 3.1 : Imprégnation de la mousse de verre avec du ruthénium(0)

### Méthode 1 :

260 µl de la suspension obtenue à l'exemple 2.1 sont imprégnés à l'aide d'une propipette ou d'une pipette Pasteur sur la mousse de verre **MV5.** La mousse est maintenue au-dessus d'un petit cristallisoir durant l'imprégnation. Quelques gouttes sont déposées sur chaque face de la mousse avec un temps de pause entre chaque dépôt. L'éventuel volume de suspension qui serait passé à travers la mousse sans y rester imprégné est récupéré dans le cristallisoir et redéposé pour effectuer une imprégnation quantitative. La mousse est ensuite séchée à l'étuve (T = 100 °C).

La mousse de verre obtenue par cette méthode est notée **Ru/MV5(I).**

### Méthode 2 :

Un morceau de mousse de verre **MV5** est immergé directement dans la suspension pendant 30 min. La mousse est ensuite séchée à l'étuve (T = 100 °C). Plusieurs cycles de trempage/séchage peuvent être effectués avant que la mousse de verre soit utilisée comme catalyseur.

La mousse de verre obtenue par cette méthode est notée **Ru/MV5(II).**

### Exemple 3.2 : Imprégnation de la mousse de verre avec du rhodium(0)

**Méthode 1 :** 2,5 cm³ de mousse de verre maintenue au-dessus d'un petit cristallisoir durant l'imprégnation (carotte d'1 cm de diamètre et de 3 cm de longueur) sont imprégnés avec 1 mL de suspension de nanoparticules de rhodium(0) préparées selon l'exemple 3.2 (C = 3,02·10⁻³ mol·L⁻¹) à l'aide d'une propipette ou d'une pipette Pasteur. Quelques gouttes sont déposées sur chaque face de la mousse avec un temps de pause entre chaque dépôt. L'éventuel volume de suspension qui serait passé à travers la mousse sans y rester imprégné est récupéré dans le cristallisoir et redéposé pour effectuer une imprégnation quantitative.

La mousse est séchée dans une étuve à 60 °C pendant deux jours.

Les mousses de verre ainsi imprégnées sont notées **Rh/MV1, Rh/MV2 et Rh/MV4.**

**Méthode 2 :** La mousse de verre **MV1,** a été imprégnée avec une suspension colloïdale de Rh(0) (1 mL, 3,02·10⁻³ mol.L⁻¹).

Cette méthode consiste à retirer les bulles d'air qui peuvent gêner le séchage uniforme de la suspension colloïdale de nanoparticules sur le support. Pour ce faire, une nouvelle technique a été développée, en mettant d'un côté du réacteur un piston et de l'autre un bouchon (Figure 2). Ainsi, en actionnant le piston, un changement de pression au niveau des pores de la mousse de verre est créé.

La mousse obtenue est dénotée **Rh/MV1(P)**

### Exemple 3.3 : Etude de l'influence du solvant sur le temps de séchage des catalyseurs :

Des suspensions de rhodium(0) ont été préparées dans les conditions de l'exemple 2.1 soit dans le méthanol.

| Entrée | Solvant | Temps de séchage (h) |
|---|---|---|
| 1 | Eau | 72 |
| 4 | MeOH | 4 |

En passant d'une préparation dans l'eau à une préparation dans le méthanol, un temps de séchage significativement plus court a pu être obtenu.

### Exemple 4 : Quantification des nanoparticules sur la mousse de l'exemple 1.2 :

1,14 cm³ de mousse de verre **MV4** est imprégnée par trempage dans 10 ml d'une suspension de nanoparticules de rhodium(0) à 3,02·10⁻³ M par immersion. Une absorbance de la solution résultante de 2,760 est mesurée à 960 nm, correspondant à une concentration en nanoparticules de 3,01·10⁻³ M. Par la loi de conservation de la matière, on en déduit une masse imprégnée de 9,21·10⁻⁴ g soit une quantité de nanoparticules déposées de 0,2 % en masse.

### Exemple 5 : Evaluation des performances de catalyseurs rhodium(0) adsorbés sur la mousse de verre dans l'hydrogénation du cyclohéxène :

L'hydrogénation catalytique du cyclohéxène est réalisée avec le montage semi-continu représenté dans la figure 1.

Le mélange H2/cyclohexène est d'abord préparé et mis en circulation dans le circuit sans passer par le réacteur de catalyse jusqu'à homogénéisation (environ 1h). Ensuite, à t=0, la réaction est démarrée (sens 2). Des prélèvements réguliers de la phase gazeuse sont effectués puis analysés pour quantifier l'évolution des concentrations de cyclohexène (CyE) et cyclohexane (CyA) au cours du temps.

Pour cela, un réservoir de 500 mL est rempli d'hydrogène pur à 1,5 bar relatif (vanne V-1 fermée), produit par un générateur d'hydrogène « H₂ Generator Alliance FDGS». La vanne V-1 est ensuite ouverte pour remplir le circuit d'hydrogène. Le circuit est ensuite purgé par V-4. Plusieurs cycles sont ainsi effectués pour rincer tout résidu dans le circuit (contrôle par GC). La pompe volumétrique à membrane (KNF) est finalement mise en route puis l'excédent d'H₂ est purgé par V-4 pour atteindre la pression atmosphérique. Le débit d'H₂ est contrôlé par un débitmètre à flotteur (Brooks, gamme ShoRate). Ensuite, 50 µL de cyclohexène pur sont introduits par une seringue de 100 µL gas-tight (SGE) par le té en verre puis volatilisé dans le flux d'H₂. L'homogénéisation du mélange est atteinte après environ 1 h de fonctionnement (contrôlé par GC).

L'hydrogénation est ensuite démarrée en permutant la vanne V-2 (sens 2) pour alimenter le réacteur. Des prélèvements réguliers (toutes les 4,5 min) sont effectués par le té en verre à l'aide d'une seringue en verre gas-tight de 500 µL (SGE). Ces prélèvements sont injectés manuellement dans un Chromatographe « Agilent technologies 6890N Network GC System ».

La fin de la réaction se traduit par la disparition du pic du CyE. La pompe est alors éteinte puis le système est purgé à l'azote.

### Détermination de la constante de vitesse de la production de cyclohexane :

Caractéristiques de la Chromatographie Phase Gaz « Agilent technologies 6890N Network GC system » utilisée :

| | | |
|---|---|---|
| Colonne : | Agilent DB - 624 | |
| | Capillaire : 30.0 m x 250 µm x 1.40 µm nominal | |
| | T = 80°C ; t_{injection} = 4.2 min | |
| | Flux : 1.3 mL/min | |
| | Vitesse moyenne = 40 cm/s | |
| Injecteur : | Split : H₂ | T = 150°C |
| | | P= 9.17 psi |
| | | Flux total = 61.7 mL/min |
| | Split ratio : 40/1 | |
| Détecteur : | T = 250°C | |
| | Flux H₂ = 40 mL/ min | |
| | Flux N₂ = 450 mL/min | |

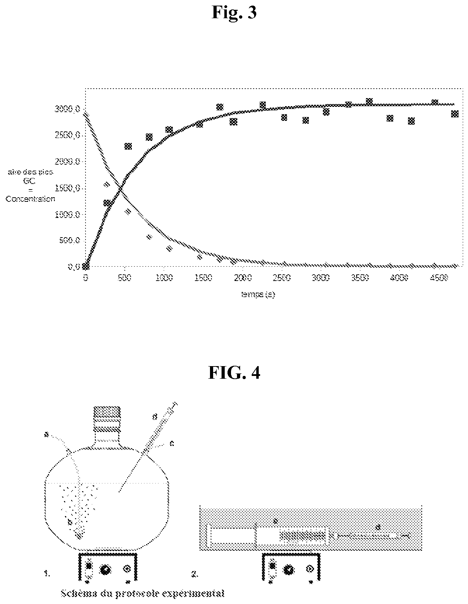

Dans ces conditions, les temps de rétention du CyA et du CyE sont respectivement de 3,45 et de 3,78 min (sans chevauchement). La calibration a été effectuée directement dans le système en injectant des quantités croissantes et connues de CyE et CyA (de 10 à 50 L) en utilisant le sens 1. Les vitesses de consommation de CyE et CyA ont été modélisées par une cinétique de 1er ordre de constante de vitesse k (en s⁻¹, figure 3): ${C}_{CyE} = {C}_{CyE}^{{t}_{0}} exp \left(-kt\right)$ ${C}_{CyA} = {C}_{CyE}^{{t}_{0}} \left(-exp\left(-kt\right)\right)$

La constante k est déterminée pour chaque expérience par résolution numérique (Solveur Excel) par la méthode des moindre-carrés. Les points expérimentaux de la concentration en CyE et CyA sont respectivement représentés par les points « carrés » et « losanges ». Les traits pleins représentent le modèle du 1er ordre. La quantification simultanée de CyE et CyA a permis de vérifier la stœchiométrie de la réaction qui était respectée à plus ou moins 10%. t_{0,5} (temps de demi-vie) et t_{0,99} (temps nécessaire pour consommer 99% du CyE) sont respectivement obtenus par les relations suivantes : ${t}_{0,5} = \frac{ln \left(2\right)}{k}$ ${t}_{0,99} = \frac{ln \left(0.01\right)}{k}$

Des mousses de verre, dont la composition est précisée dans le tableau 2, ont été préparées selon l'exemple 1 et imprégnées par une suspension de Rh(0) selon l'exemple 3. Leur activité catalytique a été testée sur la réaction d'hydrogénation du cyclohexène.

**Tableau 2. Influence des paramètres de la mousse de verre sur l'hydrogénation du cyclohexène ^{a}**

| Mousse | Porosité ouverte (%) | k^{b} (·10⁻⁴s⁻¹) | t_{1/2} ^{c} (mn) | t_{0,99} ^{c} (mn) | TOF^{d} (h⁻¹) |
|---|---|---|---|---|---|
| **MV1** | 85 | - | - | - | - |
| **Rh/MV1** | 85 | 15,52 | 7 | 49 | 200 |
| **Rh/MV2** | 83 | 11,00 | 10 | 70 | 140 |
| **Rh/MV4** | 81 | 7,12 | 16 | 108 | 90 |
| **Rh/MV1(P)** | 85 | 16,78 | 7 | 47 | 208 |
| **Rh/MV1(M)** | 85 | 15,00 | n.d. | n.d. | n.d. |
| **Rh/MV4** | 85 | 7,12 | 16 | 108 | 90 |
| **Rh/MV4** | 85 | 7,12 | 16 | 108 | 90 |

| | | | | | |
|---|---|---|---|---|---|
| Conditions: Cyclohexène (0,493 mmol, 163 éq.), Rh(0) (0.00302 mmol, 1 éq.), Substrat/Métal = 163, P(H₂) = 1atm, débitmètre à 27,6 L/h ; b : C = C₀·exp (-k·t) où C concentration de cyclohexène au temps t, C₀ concentration à t = 0s de cyclohexène et t le temps en seconde ; c : t_{1/2} = Ln (2)/k , t_{0,99} = - Ln (0,01)/k ; d : TOF = Nombre de moles de substrat converti/ (Nombre de moles de Rh * unité de temps). | | | | | |

De cette étude, il apparaît que la mousse de verre imprégnée avec du rhodium(0) est active pour la conversion du cyclohexène en cyclohexane en phase gaz, alors qu'aucune activité n'est observée pour une mousse de verre non dopée (test blanc). Les particules de Rh(0) constituent donc les espèces actives pour cette réaction.

En passant d'une préparation dans l'eau à une préparation dans le méthanol **(Rh/MV1** *vs* **Rh/MV1(M)),** aucune différence d'activité catalytique n'est observée entre les deux matériaux (k d'environ 15·10⁻⁴ s⁻¹).

Quelle que soit la méthode d'imprégnation utilisée **(Rh/MV1** *vs* **Rh/MV1(P)),** une activité catalytique comparable est observée entre les deux matériaux.

### Exemple 6 : Stabilité des matériaux et recyclabilité

### Exemple 6.1 : Stabilité des nanoparticules sur la mousse de verre

Des essais de lixiviation par Soxhlet (eau) sur 24h ont été menés sur :
a) une mousse de verre non imprégnée (témoin),
b) une mousse de verre imprégnée avec des nanoparticules de ruthénum(0),
c) une mousse de verre imprégnée avec des nanoparticules de rhodium(0).

Après 24 heures, il n'y a pas de changement de couleur des différentes mousses de verre, ni de coloration de la solution de lessivage.

Les nanoparticules restent donc adsorbées sur le support en mousse de verre, prouvant la stabilité vis-à-vis de la lixiviation de ces mousses de verre imprégnées.

### Exemple 6.2 : Stabilité des nanoparticules sur la mousse de verre

### Mode opératoire des réactions de recyclage :

Après avoir testé la mousse **Rh/MV1** pour l'hydrogénation catalytique, plusieurs réactions de recyclage ont été réalisées. Le premier recyclage est réalisé dans les mêmes conditions opératoires que la réaction initiale (Exemple 5 : 50 µL de cyclohexène, 0,493 mmol, débit à 27,6L/h). Puis, quand la réaction de Recyclage 1 se termine, tout le circuit est purgé à l'azote pendant 30 minutes. La partie réacteur (mousse + catalyseur) est mise sous air comprimé. Une fois le circuit totalement purgé, la réaction est refaite à l'identique (50 µL de cyclohexène, 0,493 mmol, débit à 27,6L/h). Ces étapes de purge et de réaction sont répétées autant de fois que de recyclages faits. La cinétique de chaque réaction est ensuite analysée. Les résultats sont reportés dans le tableau 3 :

**Tableau 3 :**

| Entrée | Réactions^{a} | k^{b}(·10⁻⁴ s⁻¹) | t_{½} ^{c}(mn) | t_{0.99} ^{c} (mn) | TOF ^{d} (h⁻¹) |
|---|---|---|---|---|---|
| 1 | Test de reproductibilité | 15,45 | 7 | 50 | 196 |
| 2 | Test de référence | 15,52 | 7 | 49 | 200 |
| 3 | Recyclage 1 | 21,47 | 5 | 36 | 272 |
| 4 | Recyclage 2 | 18,11 | 6 | 42 | 233 |
| 5 | Recyclage 3 | 14,55 | 8 | 53 | 184 |

| | | | | | |
|---|---|---|---|---|---|
| A : Cyclohexène (0,493 mmol, 163 éq.), Rh(0) (0.00302 mmol, 1 éq.), Substrat/Métal = 163, P(H₂) = 1 atm, Débitmètre à 27,6 L/h ; B : C = C₀·exp (-k·t) où C concentration de cyclohexène au temps t, C₀ concentration à t = 0s de cyclohexène et t le temps en seconde ; C : t_{1/2} = Ln (2)/k, t_{0,99} = - Ln (0,01)/k ; D : TOF = Nombre de moles de cyclohexène converti/ (Nombre de moles de Rh * unité de temps). | | | | | |

La synthèse d'un catalyseur **Rh/MV1** a été réalisée de manière reproductible (Entrée 1 vs Entrée 2) sur la base d'une mousse de même composition et de porosité ouverte similaire. De plus, une activité catalytique similaire, avec une constante de vitesse voisine de 15,5·10⁻⁴ s⁻¹, a été obtenue, validant ainsi la reproductibilité de la méthode d'imprégnation des nanoparticules de rhodium(0) sur la mousse de verre.

Par ailleurs, une activité comparable (à l'erreur expérimentale près) est observée avec le matériau **Rh/MV1** sur quatre cycles (Entrées 2-5).

### Exemple 7 : Ozonation catalytique

### Protocole expérimental

Pour se concentrer sur le devenir de l'ozone dissous et de la formation de radicaux hydroxyles dans l'eau, le transfert gaz/liquide de l'ozone et la réaction ont été séparés. Dans le cadre d'une production industrielle ces deux étapes seront rassemblées.

Dans un flacon fermé de 2L, l'ozone est dissous dans 2 ml d'eau acidifiée par de l'acide sulfurique 1N (Figure 4). L'ozone gazeux produit par l'ozoneur rentre par un tube coudé (a) et est dispersé par un poreux (b). Les évents sortent par le tube coudé (c) et sont directement envoyés vers le destructeur. Une seringue de 10 mL (d) est utilisée pour prélever l'ozone dissous dans le flacon de 2L une fois que la concentration d'ozone a atteint un régime stationnaire aux alentours de 130 g/Nm³.

Cette solution est ensuite injectée dans une seringue de 100 ml (e) hermétique aux gaz (qui joue le rôle d'un réacteur fermé « batch ») contenant environ 90 ml d'eau à analyser (eau ozonée-déozonée dans un premier temps), le traceur de radicaux et la mousse de verre. C'est à ce moment que la réaction commence (t0).

Le traceur de radicaux utilisé est le *p*CBA (acide *para*-chlorobenzoique) en raison de sa réactivité. La cinétique de décomposition de l'ozone et de formation de radicaux sera étudiée avec différentes concentrations de *p*CBA et différentes mousses de verre.

L'utilisation d'une seringue comme réacteur permet de réaliser des prélèvements sans générer d'espaces de têtes.

Les prélèvements ont permis de réaliser un suivi temporel des concentrations d'ozone dans le réacteur grâce à la méthode du carmin d'indigo. La quantité de radicaux hydroxyles formés est obtenue après l'analyse temporelle de la concentration en *p*CBA qui sera réalisée par UPLC.

Test au Carmin d'indigo (Hoigné et Bader) : La concentration en ozone dissous dans le réacteur peut être déterminée simplement et rapidement au cours du temps grâce à la méthode du carmin d'indigo qui permet de quantifier des concentrations se situant entre 10 µg/L et 20 mg/L. La réaction entre l'ozone et l'indigo trisulfonate est stœchiométrique et rapide en milieu acide. Cette réaction provoque une décoloration de l'indigo trisulfonate et donc une diminution de l'absorbance. Cette diminution est proportionnelle à la quantité d'ozone dissous. La molécule d'indigo trisulfonate absorbe à 600 nm, la mesure de l'absorbance à cette longueur d'onde permet donc de quantifier l'ozone présent dans le réacteur au cours du temps.

La concentration en ozone dans le réacteur [O₃], en mg/L, est déterminée grâce à l'équation suivante : $\left[{O}_{3}\right] = \left({V}_{T}.\left({A}_{blanc}-{A}_{ech}\right)\right) / \left(f.{V}_{E}.1\right)$ V_{T} représente le volume total (ml) tandis que V_{E} correspond au volume de l'échantillon (ml). f représente la perte d'absorbance de l'indigo par mg/L d'ozone (0,42 par mg/L) et le trajet optique de la cuve (cm).

Dans cette formule l'absorbance du blanc est considérée sur une solution préparée dans les mêmes conditions que l'échantillon. Or ce n'est pas le cas, on réalise alors une extrapolation à partir de l'absorbance mesurée de la solution d'indigo II diluée 25/75 : ${A}_{blanc} = 4 . {A}_{ind} . {V}_{ind} / {V}_{T}$
V_{ind} : Volume d'indigo II dans l'échantillon (mL)
V_{T} : Volume total de l'échantillon (mL)

### Méthode :

Pour réaliser ce test au carmin d'indigo il faut préalablement avoir injecté 5 mL de solution d'indigo II dans un flacon ambré. Autant de flacons que de prélèvements seront utilisés. Ces flacons sont dans un premier temps pesé à vide puis après l'injection des 5 mL pour connaitre le volume exacte contenu dans le flacon.

Dans trois autres flacons, environ 35 mL de la solution d'indigo II sont injectés pour déterminer la concentration en ozone qui sera injectée dans le réacteur.

Un prélèvement précis de 10 mL d'eau ozonée est injecté dans la seringue de 100 mL qui sert de réacteur. Sont ensuite réalisés des prélèvements réguliers (entre 5 et 10 mL environ) dans le réacteur durant un temps variable. En effet le temps de manipulation est très variable selon la présence ou non de mousse et selon que cette mousse est imprégnée au Ru(0) ou non. Ces prélèvements sont injectés dans les flacons.

A la fin de la manipulation tous les flacons sont pesés de nouveau pour connaître la quantité exacte d'eau injectée. La mesure de l'absorbance à 600 nm permet de déterminer la concentration en ozone dans le flacon et donc dans le temps. Un petit volume de chaque flacon est conservé puis injecté dans des piluliers pour analyser la teneur en pCBA.

### Analyse du pCBA :

L'analyse du *p*CBA permet de déterminer la concentration en radicaux hydroxyle HO^{.} formés dans le réacteur au cours du temps. Le *p*CBA est utilisé comme traceur de radicaux car il possède une constante de vitesse élevée avec les radicaux HO^{.} en milieu acide (k = 5·10⁹ L·mol⁻¹·s⁻¹) et sa réaction avec l'ozone est négligeable (0,15 L·mol⁻¹·s⁻¹). La réaction entre l'ozone et le carmin d'indigo permet de figer la décomposition de l'ozone et la concentration en pCBA.

L'analyse est réalisée par UPLC-UV à 237 nm. 50 µl de l'échantillon, préalablement filtré sur un filtre de porosité 0,2 µm sont injectés. L'analyse dure 3 min.

### Exemple 7.1 : Cinétique de décomposition de l'ozone

Cinq mélanges différents (mélanges A à E) sont testés dans le réacteur :
*Mélange A :* 90 ml eau ozonée-déozonée + 1 ml *p*CBA (200 ug/L) + 10 ml eau ozonée,
*Mélange B* : 90 ml eau ozonée-déozonée + 1 ml *p*CBA (200 ug/L) + 10 ml eau ozonée + 1 g de mousse non imprégnée **MV5,**
*Mélange C :* 90 ml eau ozonée-déozonée + 1 ml *p*CBA (200 ug/L) + 10 ml eau ozonée + 1g de mousse imprégnée **Ru/MV5(I),**
*Mélange D :* 90 ml eau ozonée-déozonée + 2 ml *p*CBA (400 ug/L) + 10 ml eau ozonée + 350 mg de mousse imprégnée **Ru/MV5(I),**
*Mélange E :* 90 ml eau ozonée-déozonée + 2 mL *p*CBA (400 ug/L) + 10 ml eau ozonée + 350 mg de mousse saturée **Ru/MV5(II)**

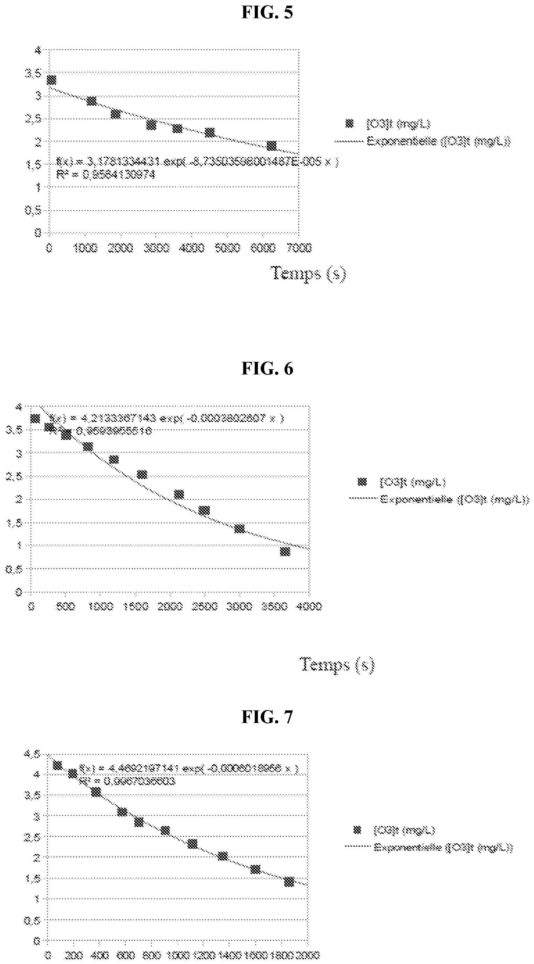

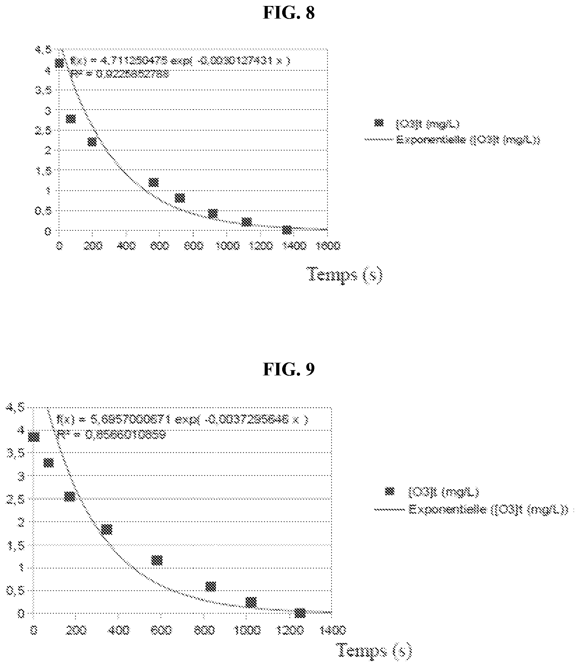

Les courbes obtenues sont représentées dans les figures 5 à 9 et les temps de demi-vie de l'ozone dans le tableau X. On assimile les courbes obtenues à des cinétiques du 1er ordre, soit : $ln {\left[{O}_{3}\right]}_{0} / {\left[{O}_{3}\right]}_{t} = {k}_{obs} ⋅ t$

On obtient ainsi le temps de demi-vie de l'ozone d'après la formule suivante : ${t}_{1 / 2} = \left(ln 2\right) / {k}_{obs} .$

| | Mélange A | Mélange B | Mélange C | Mélange D | Mélange E |
|---|---|---|---|---|---|
| k_{obs} | 8,7·10⁻⁵ | 3,8.10⁻⁴ | 6·10⁻⁴ | - | - |
| t_{1/2} (s) | 7900 | 1800 | 1150 | 500 | 400 |

La cinétique de décomposition de l'ozone est fortement marquée par la présence de la mousse de verre imprégnée. (**Figure 5** comparée aux **Figures 6** à **9**).

Ces manipulations permettent de démontrer l'efficacité de la mousse de verre imprégnée sur la décomposition de l'ozone. En effet, la présence d'une mousse non imprégnée a permis de diviser le temps de demi-vie de l'ozone dans le réacteur par trois. L'imprégnation a permis de diviser une nouvelle fois celui-ci par deux.

Les mélanges D et E montrent qu'à première vue la quantité de ruthénium présente sur la mousse ne joue pas un rôle très important, puisque un temps de demi-vie similaire est observé pour une mousse imprégnée et une mousse saturée.

Les temps de demi-vie de l'ozone sont, en apparence, très différents selon la masse de la mousse. Il semblerait que ce phénomène ne soit pas dû en réalité à la masse de mousse utilisée mais plutôt à sa surface spécifique.

### Exemple 7.2 : Génération de radicaux

Les manipulations effectuées sont les mêmes que précédemment (Exemple 7.1), les analyses d'ozone et de *p*CBA pouvant être effectuée simultanément.

Une manipulation supplémentaire a été effectuée pour étudier l'adsorption du *p*CBA sur le nanocatalyseur en absence d'ozone avec un *Mélange F :* 90 ml eau ozonée-déozonée + 1 ml *p*CBA (200 ug/L) + 10 ml eau ultra-pure acidifiée.

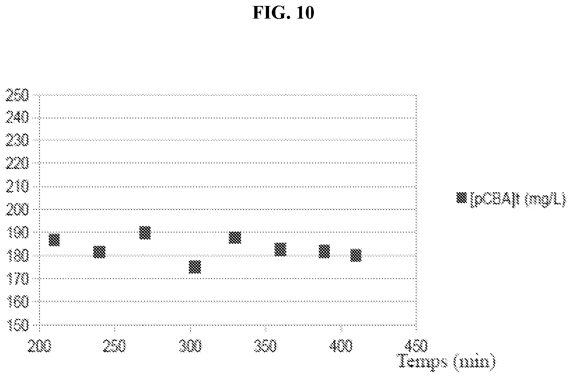

Les résultats sont présentés dans la figure 10.

Cette manipulation a permis de démontrer que l'adsorption du *p*CBA sur la mousse de verre est négligeable. Après 6h, la concentration en *p*CBA reste proche des 200 µg/L de départ (Figure 10).

### Exemple 8 : Réactions d'oxydation par l'ozone

La mousse de verre utilisée dans cet exemple est préparée par traitement thermique à 850 °C pendant 2 heures d'un mélange constitué de 95.5% de billes de verres, 1.5% AlN et 3% TiO₂.

### Exemple 8.1 : Réaction d'oxydation du toluène avec un catalyseur au ruthénium

**Conditions :** La mousse a été imprégnée sur les deux faces par 5 mL (∼1,9·10⁻⁵ mol) d'une solution de nanoparticules de Ruthénium selon l'exemple 2.1.

10 µL de toluène ont été injectés dans le circuit de réaction. Concentration O₃ - environ 70-80 g/Nm³

**Résultats :** On observe une diminution significative du pic du toluène au bout de 5 min en chromatographie en phase gazeuse. Le pic continue de diminuer lors des injections suivantes. Après 45 min, on n'observe plus d'évolution, la réaction est donc arrêtée.

### Exemple 8.2 : Réaction d'oxydations d'isopropanol

Conditions : le protocole est identique à celui de l'exemple 8.1.

**Résultats** : Sur les chromatogrammes des 2 premières injections, on observe la diminution de pic d'isopropanol et l'apparition du pic d'acétone. On observe ensuite une diminution du pic d'acétone qui se stabilise au bout de 40-45 min de réaction.

**Exemple 8.3 : Réaction d'oxydations d'isopropanol et du toluène avec un catalyseur au rhodium**

**Conditions :** Les tests sont identiques à ceux des exemples 8.1 et 8.2. la mousse de verre est imprégnée des deux côtés avec 5 mL d'une solution de rhodium selon l'exemple 2.2.

**Résultats :** On observe, comme avec le catalyseur au ruthénium une diminution du pic de toluène et du pic de l'isopropanol. La réaction est cependant plus lente qu'avec le catalyseur au ruthénium.

### Conclusions:

Les mousses imprégnées avec du ruthénium ou du rhodium sont actives dans les réactions d'oxydation du toluène ou de l'isopropanol. Le catalyseur au ruthénium reste le plus actif dans ces réactions.

## Revendications

1. Matériau comprenant :
(a) un support en mousse de verre,
(b) des nanoparticules métalliques constituées à au moins 90 % d'un métal à l'état d'oxydation 0 directement adsorbées sur ledit support,
- ladite mousse de verre présentant une porosité ouverte d'au moins 75 % par rapport à la porosité totale, avantageusement d'au moins 80 %, de préférence au moins 85 %,
- ledit matériau ne comprenant pas de couche d'enduction entre la mousse de verre et les nanoparticules métalliques,
- ladite mousse de verre étant amorphe ou partiellement cristallisé, la cristallinité étant inférieure à 20 %, déterminée par la diffraction aux rayons X
- ladite mousse de verre ayant une masse volumique de 0,15 à 0,8 g/cm³,
- ladite porosité ouverte étant déterminée selon l'équation : ${%P}_{ouverte} = \left(1-\frac{{ρ}_{app}}{{ρ}_{pyc}}\right) \times 100 ,$ dans laquelle :
*ρₐₚₚ* est la masse volumique apparente déterminée par le ratio entre la masse de la mousse et son volume total incluant les pores
*ρ_{pyc}* est la masse volumique mesurée par pycnométrie à l'hélium,
dans lequel le diamètre moyen des pores de la mousse de verre dₚ est de 0,01 mm à 1 mm, avantageusement de 0,2 à 0,8 mm, notamment de 0,4 à 0,7 mm, déterminé par traitement d'images de la surface de la mousse de verre, en particulier par le logiciel Image J.

2. Matériau selon la revendication 1, dans lequel la mousse de verre a une porosité totale d'au moins 60 %, avantageusement d'au moins 70 %, plus avantageusement d'au moins 75 % et de préférence d'au moins 80 %.

3. Matériau selon l'une des revendications là 2, dans lequel le métal est un métal de transition du groupe IB, IIB, VA, IVB, VB, VIB, VIIB ou VIIIB.

4. Matériau selon la revendication 3, dans lequel le métal est choisi dans le groupe constitué de Fe, Co, Ni, Pd, Ru, Rh, Au, Pt, Cu, Ag, Bi, Re, Mo, Ir, V, Cd, et Zn.

5. Matériau selon l'une des revendications 1 à 4, dans lequel les nanoparticules ont un diamètre moyen de 1 à 10 nm, avantageusement de 2 à 8 nm, de préférence environ 5 nm, déterminé par traitement d'une image obtenue par microscopie électronique à balayage ou à transmission d'une suspension de nanoparticules dans un solvant, de préférence par ImageJ ou par le logiciel SCION.

6. Procédé de préparation d'un matériau comprenant :
a. un support en mousse de verre,
b. des nanoparticules métalliques constituées à au moins 90 % d'un métal à l'état d'oxydation 0 directement adsorbées sur ledit support,
comprenant une étape de mise en contact de nanoparticules métalliques constituées à au moins 90 % d'un métal à l'état d'oxydation 0 dont le métal est à l'état d'oxydation 0 en suspension dans un solvant avec une mousse de verre, et optionnellement une étape de séchage du matériau,
ledit matériau ne comprenant pas de couche d'enduction entre la mousse de verre et les nanoparticules métalliques..

7. Procédé selon la revendication 6, dans lequel la suspension comprend un agent stabilisant notamment choisi parmi les polymères, les liquides ioniques, les ligands phosphorés et/ou soufrés et/ou azotés et/ou oxygénés, les cyclodextrines, les dendrimères, les calixarènes et les tensioactifs.

8. Procédé selon la revendication 6 dans lequel le solvant est l'eau, le méthanol ou un mélange de ces deux solvants.

9. Procédé selon la revendication 7, dans lequel l'agent stabilisant est un tensioactif de type ammonium quaternaire, de préférence un N,N-diméthyl-N-cétyl-N-(2-hydroxyéthyl) ammonium.

10. Réacteur catalytique comprenant un matériau tel que défini à l'une des revendications 1 à 5.

11. Utilisation d'un matériau selon l'une des revendications 1 à 5, dans la dégradation de polluants en présence d'ozone, les réactions de réductions en phase gazeuse en présence d'H₂ telles que l'hydrogénation d'alcènes, d'alcynes ou d'arènes et la réaction tandem déshalogénation/hydrogénation, les réactions d'oxydations en phase gazeuse en présence d'O₂, la préparation d'ammoniac NH₃ en phase gazeuse à partir de N₂ et de H₂, les réactions en phase gazeuse en présence de CO, telle que la réaction de Fischer-Tropsch et les réactions de carbonylations, les réactions de carboxylations en phase gazeuse en présence de CO₂, ou la décomposition du N₂O en N₂, éventuellement en présence de H₂ ou CO.

12. Utilisation selon la revendication 11 pour l'hydrogénation d'alcènes, d'alcynes ou d'arènes et la réaction tandem déshalogénation/hydrogénation, d'un matériau selon l'une des revendications 1 à 5 comprenant avantageusement des nanoparticules de Rh(0), de Pd(0) ou de Pt(0), de préférence de Rh(0), ou d'un réacteur catalytique selon la revendication 10.

## Patentansprüche

1. Material, umfassend:
(a) einen Träger aus Glasschaum,
(b) metallische Nanopartikel, die aus mindestens 90 % eines Metalls im Oxidationszustand 0 bestehen und direkt auf dem genannten Träger adsorbiert sind,
- wobei der genannte Glasschaum eine offene Porosität von mindestens 75 %, bezogen auf die gesamte Porosität, vorteilhaft von mindestens 80 %, vorzugsweise mindestens 85 %, aufweist,
- wobei das genannte Material keine Verteilungsschicht zwischen dem Glasschaum und den metallischen Nanopartikeln umfasst,
- wobei der genannte Glasschaum amorph oder teilweise kristallisiert ist, wobei die Kristallinität kleiner ist als 20 %, bestimmt durch Röntgenstrahlbeugung,
- wobei der genannte Glasschaum eine Volumenmasse von 0,15 bis 0,8 g/cm³ aufweist,
- wobei die genannte offene Porosität bestimmt wird gemäß der Gleichung: ${%P}_{ouverte} = \left(1-\frac{{ρ}_{app}}{{ρ}_{pyc}}\right) \times 100 ,$ wobei:
ρₐₚₚ die scheinbare Volumenmasse ist, bestimmt durch das Verhältnis zwischen der Masse des Schaums und seinem gesamten Volumen einschließlich der Poren,
ρ_{pyc} die Volumenmasse ist, gemessen durch Pyknometrie mit Helium,
wobei der mittlere Durchmesser der Poren des Glasschaums dₚ von 0,01 mm bis 1 mm, vorteilhaft von 0,2 bis 0,8 mm, insbesondere von 0,4 bis 0,7 mm, beträgt, bestimmt durch Bildbearbeitung der Oberfläche des Glasschaums, insbesondere durch die Software Image J.

2. Material nach Anspruch 1, wobei der Glasschaum eine gesamte Porosität von mindestens 60 %, vorteilhaft von mindestens 70 %, vorteilhafter von mindestens 75 % und vorzugsweise von mindestens 80 %, aufweist.

3. Material nach einem der Ansprüche 1 bis 2, wobei das Metall ein Übergangsmetall der Gruppe IB, IIB, VA, IVB, VB, VIB, VIIB oder VIIIB ist.

4. Material nach Anspruch 3, wobei das Metall ausgewählt ist aus der Gruppe bestehend aus Fe, Co, Ni, Pd, Ru, Rh, Au, Pt, Cu, Ag, Bi, Re, Mo, Ir, V, Cd und Zn.

5. Material nach einem der Ansprüche 1 bis 4, wobei die Nanopartikel einen mittleren Durchmesser von 1 bis 10 nm, vorteilhaft von 2 bis 8 nm, vorzugsweise ungefähr 5 nm, aufweisen, bestimmt durch Bearbeitung eines Bilds, das durch Raster- oder Transmissionselektronenmikroskopie einer Suspension von Nanopartikeln in einem Lösungsmittel erhalten wird, vorzugsweise durch Image J oder durch die Software SCION.

6. Verfahren zur Herstellung eines Materials, umfassend:
a. einen Träger aus Glasschaum,
b. metallische Nanopartikel, die aus mindestens 90 % eines Metalls im Oxidationszustand 0 bestehen und direkt auf dem genannten Träger adsorbiert sind,
umfassend einen Schritt des Inberührungbringens der metallischen Nanopartikel, die aus mindestens 90 % eines Metalls im Oxidationszustand 0 bestehen, wobei das Metall im Oxidationszustand 0 in Suspension in einem Lösungsmittel mit einem Glasschaum ist, und gegebenenfalls einen Schritt des Trocknens des Materials,
wobei das genannte Material keine Verteilungsschicht zwischen dem Glasschaum und den metallischen Nanopartikeln umfasst.

7. Verfahren nach Anspruch 6, wobei die Suspension ein Stabilisierungsmittel umfasst, ausgewählt aus Polymeren, ionischen Flüssigkeiten, phosphorhaltigen und/oder schwefelhaltigen und/oder stickstoffhaltigen und/oder sauerstoffhaltigen Liganden, Cyclodextrinen, Dendrimeren, Calixarenen und grenzflächenaktiven Stoffen.

8. Verfahren nach Anspruch 6, wobei das Lösungsmittel Wasser, Methanol oder eine Mischung dieser beiden Lösungsmittel ist.

9. Verfahren nach Anspruch 7, wobei das Stabilisierungsmittel ein grenzflächenaktiver Stoff vom Typ eines quaternären Ammoniums ist, vorzugsweise N,N-Dimethyl-N-cetyl-N-(2-hydroxyethyl)-ammonium.

10. Katalytischer Reaktor, umfassend ein Material wie in einem der Ansprüche 1 bis 5 definiert.

11. Verwendung eines Materials nach einem der Ansprüche 1 bis 5, beim Abbau von Verunreinigungen in Anwesenheit von Ozon, in Reduktionsreaktionen in der Gasphase in Anwesenheit von H₂, wie der Hydrierung von Alkenen, von Alcynen oder von Arenen, und der Tandem-Reaktion Dehalogenierung/Hydrierung, Oxidationsreaktionen in der Gasphase in Anwesenheit von O₂, bei der Herstellung von Ammoniak NH₃ in der Gasphase aus N₂ und aus H₂, in Reaktionen in der Gasphase in Anwesenheit von CO, wie der Fischer-Tropsch-Reaktion und Carbonylierungsreaktionen, Carboxylierungsreaktionen in der Gasphase in Anwesenheit von CO₂, oder bei der Zersetzung von N₂O zu N₂, gegebenenfalls in Anwesenheit von H₂ oder CO.

12. Verwendung nach Anspruch 11, für die Hydrierung von Alkenen, von Alcynen oder von Arenen und die Tandem-Reaktion Dehalogenierung/Hydrierung, wobei eines Materials nach einem der Ansprüche 1 bis 5 vorteilhaft Nanopartikel von Rh(0), von Pd(0) oder von Pt(0), vorzugsweise von Rh(0), oder von einem katalytischen Reaktor nach Anspruch 10 umfasst.

## Claims

1. Material comprising:
(a) a glass foam support,
(b) metal nanoparticles in which at least 90% are constituted by a metal at oxidation state 0 directly adsorbed on said support,
- said glass foam having an open porosity of at least 75% with respect to the total porosity, advantageously at least 80%, preferably at least 85%,
- said material being devoid of a washcoat between the glass foam and the metal nanoparticles,
- said glass foam being amorphous or partially crystallized, the crystallinity being less than 20%, determined by X-ray diffraction,
- said glass foam having a density form 0.15 to 0.8 g/cm³,
- said open porosity of the glass foam being determined according to the equation: ${%P}_{open} = \left(1-\frac{{ρ}_{app}}{{ρ}_{pyc}}\right) \times 100 ,$ in which:
*ρₐₚₚ* is the apparent density determined by the ratio between the mass of the foam and its total volume including the pores
*ρ_{pyc}* is the density measured by helium pycnometry,
in said material the mean diameter of the pores of the glass foam dₚ is from 0.01 mm to 1 mm, advantageously 0.2 to 0.8 mm, in particular 0.4 to 0.7 mm, determined by image processing of the surface of a glass foam, in particular carried out with the ImageJ software.

2. Material according to one of claims 1, in which the glass foam has a total porosity of at least 60%, advantageously at least 70%, more advantageously at least 75%, and preferably at least 80%.

3. Material according to one of claims 1 to 2, in which the metal is a transition metal of Group IB, IIB, VA, IVB, VB, VIB, VIIB or VIIIB.

4. Material according to claim 3, in which the metal is selected from the group constituted by Fe, Co, Ni, Pd, Ru, Rh, Au, Pt, Cu, Ag, Bi, Re, Mo, Ir, V, Cd, and Zn.

5. Material according to one of claims 1 to 4, in which the nanoparticles have a mean diameter of 1 to 10 nm, advantageously 2 to 8 nm, preferably approximately 5 nm, determined by image processing of the image obtained by scanning electron microscopy or by transmission electron microscopy of a suspension of metal nanoparticles in a solvent, preferably carried out with the ImageJ software or SCION software.

6. Process for the preparation of a material comprising:
a. a glass foam support,
b. metal nanoparticles in which at least 90% are constituted by a metal at oxidation state 0 directly adsorbed on said support,
comprising a step of contacting metal nanoparticles in which at least 90% are constituted by a metal at oxidation state 0 the metal of which is at oxidation state 0 in suspension in a solvent with a glass foam, and optionally comprising a step of drying the material,
said material being devoid of a washcoat between the glass foam and the metal nanoparticles.

7. Process according to claim 6, in which the suspension comprises a stabilizing agent selected in particular from polymers, ionic liquids, phosphorous-containing and/or sulphur-containing and/or nitrogen-containing and/or oxygen-containing ligands, cyclodexdrins, dendrimers, calixarenes and surfactants.

8. Process according to claim 6 in which the solvent is water, methanol or a mixture of these two solvents.

9. Process according to claim 7, in which the stabilizing agent is a surfactant of the quaternary ammonium type, preferably an N,N-dimethyl-N-cetyl-N-(2-hydroxyethyl) ammonium.

10. Catalytic reactor comprising a material as defined in one of claims 1 to 5.

11. Use of a material according to one of claims 1 to 5, in the degradation of pollutants in the presence of ozone, gas phase reduction reactions in the presence of H₂ such as hydrogenation of alkenes, alkynes or arenes and the tandem dehalogenation/hydrogenation reaction, gas phase oxidation reactions in the presence of O₂, gas phase preparation of ammonia NH₃ from N₂ and H₂, gas phase reactions in the presence of CO, such as the Fischer-Tropsch reaction and the carbonylation reactions, gas phase carboxylation reactions in the presence of CO₂ or the decomposition of N₂O to N₂, optionally in the presence of H₂ or CO.

12. Use according to claim 11 for the hydrogenation of alkenes, alkynes or arenes and the tandem dehalogenation/hydrogenation reaction, of a material according to one of claims 1 to 5 advantageously comprising nanoparticles of Rh(0), Pd(0) or Pt(0), preferably Rh(0), or of a catalytic reactor according to claim 10.
